(19) Europäisches Patentamt
European Patent Office
Office européen des brevets

(11) **EP 4 243 750 B1**

(12) **EUROPEAN PATENT SPECIFICATION**

(45) Date of publication and mention
of the grant of the patent:
**23.07.2025 Bulletin 2025/30**

(21) Application number: **21802080.8**

(22) Date of filing: **06.10.2021**

(51) International Patent Classification (IPC):
*A61F 13/514* (2006.01)      *A61F 13/64* (2006.01)

(52) Cooperative Patent Classification (CPC):
**A61F 13/51478; A61F 13/51498; A61F 13/64**

(86) International application number:
**PCT/US2021/053705**

(87) International publication number:
**WO 2022/103524 (19.05.2022 Gazette 2022/20)**

(54) **BELT ASSEMBLIES FOR ABSORBENT ARTICLES**

GÜRTELANORDNUNGEN FÜR ABSORBIERENDE ARTIKEL

ENSEMBLES CEINTURE POUR ARTICLES ABSORBANTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.11.2020 US 202063111790 P**

(43) Date of publication of application:
**20.09.2023 Bulletin 2023/38**

(60) Divisional application:
**25182900.8**

(73) Proprietor: **The Procter & Gamble Company
Cincinnati, OH 45202 (US)**

(72) Inventors:
• **TONG, Ling
Beijing 101312 (CN)**
• **ROE, Donald, Carroll
Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
WO-A1-2016/122629      CN-A- 103 079 514
US-A1- 2018 168 874      US-A1- 2019 070 042

**Description**

FIELD OF THE INVENTION

The present disclosure relates to absorbent articles including belts, and more particularly, to front and back waist belt assemblies.

BACKGROUND OF THE INVENTION

Along an assembly line, various types of articles, such as for example, diapers and other absorbent articles, may be assembled by adding components to and/or otherwise modifying an advancing, continuous web of material. For example, in some processes, advancing webs of material are combined with other advancing webs of material. In other examples, individual components created from advancing webs of material are combined with advancing webs of material, which in turn, are then combined with other advancing webs of material. In some cases, individual components created from an advancing web or webs are combined with other individual components created from other advancing webs. Webs of material and component parts used to manufacture diapers may include: backsheets, topsheets, leg cuffs, waist bands, absorbent core components, front and/or back ears, fastening components, and various types of elastic webs and components such as leg elastics, barrier leg cuff elastics, stretch side panels, and waist elastics. Once the desired component parts are assembled, the advancing web(s) and component parts are subjected to a final knife cut to separate the web(s) into discrete diapers or other absorbent articles.

Some absorbent articles have components that include elastomeric laminates. Such elastomeric laminates may include an elastic material bonded to one or more nonwovens. The elastic material may include an elastic film and/or elastic strands. In some laminates, a plurality of elastic strands are joined to a nonwoven while the plurality of strands are in a stretched condition so that when the elastic strands relax, the nonwoven gathers between the locations where the nonwoven is bonded to the elastic strands, and in turn, forms corrugations and rugosities. The resulting elastomeric laminate is stretchable to the extent that the corrugations allow the elastic strands to elongate.

Stranded elastomeric laminates are sometimes used to make diaper pant belts. In some instances, it may be desirable to provide diaper pant belts with features that may differ along a width and/or length of the belts. Such features may include, for example, belt tensioning, rugosities, bonding patterns, and aperture arrangements. However, imparting such varying features to localized regions of an elastic laminate can result in various challenges relating to web handling and quality challenges during the absorbent article assembly process.

Consequently, it would be beneficial to provide elastic laminates for use as diaper belts having various features that differ along a width and/or length of the laminates while at the same time having a construction that reduces some of the difficulties associated with the assembly process that may otherwise be associated with imparting such features to an assembled laminate.

US 2018168874 relates to absorbent articles that may comprise one or more pluralities of elastics which are spaced by less than 4 mm, have less than 300 dtex and/or less than 300%, strain to deliver a pressure less than 1 psi under the elastics, while providing a modulus of between about 2 gf/mm and 15 gf/mm.

SUMMARY OF THE INVENTION

The invention is defined in claim 1. Optional features are found in the dependent claims.

An absorbent article comprising: a chassis comprising a topsheet, a backsheet, and an absorbent core positioned between the topsheet and the backsheet, the chassis further comprising a first end region and a second end region longitudinally separated from the first end region by a crotch region; a first belt connected with the first end region of the chassis; a second belt connected with the second end region of the chassis, wherein laterally opposing end portions of the second belt are connected with laterally opposing end portions of the first belt to form a waist opening; wherein at least one of the first belt and the second belt comprises: an elastic material positioned between and connected with a first substrate and a second substrate; wherein the first substrate and the second substrate each comprise a wearer facing surface, a garment facing surface, and a distal edge extending along a portion of the waist opening; and a panel layer comprising a third substrate connected with the garment surface of the first substrate and the wearer facing surface of the second substrate, the panel layer folded along a fold line that defines at least a portion of the waist opening.

An absorbent article comprising: a chassis comprising a topsheet, a backsheet, and an absorbent core positioned between the topsheet and the backsheet, the chassis further comprising a first end region and a second end region longitudinally separated from the first end region by a crotch region; a first belt connected with the first end region of the chassis; a second belt connected with the second end region of the chassis, wherein laterally opposing end portions of the second belt are connected with laterally opposing end portions of the first belt to form a waist opening; wherein at least one of the first belt and the second belt comprises: an elastic material positioned between and connected with a first substrate and a second substrate; wherein at least one of the first substrate and the second substrate comprises a distal edge extending along a portion of the waist opening; and a panel layer extending longitudinally from a first lateral edge to a second lateral edge, the panel layer connected with at least one of the first substrate and the second substrate; a first region that comprises the panel layer; a second region outside the first region that does not comprise the panel layer; and wherein when the elastic material is contracted, the first region and the second region each comprise longitudinally extending gathers, wherein the first region comprises a first Rugosity Frequency and a first Rugosity Wavelength, and the second region comprises a second Rugosity Frequency and a second Rugosity Wavelength; and wherein the first Rugosity Frequency is not equal to the second Rugosity Frequency.

An absorbent article comprising: a chassis comprising a topsheet, a backsheet, and an absorbent core positioned between the topsheet and the backsheet, the chassis further comprising a first end region and a second end region longitudinally separated from the first end region by a crotch region; a first belt connected with the first end region of the chassis; a second belt connected with the second end region of the chassis, wherein laterally opposing end portions of the second belt are connected with laterally opposing end portions of the first belt to form a waist opening; wherein at least one of the first belt and the second belt comprises: an elastic material positioned between and connected with a first substrate and a second substrate; wherein at least one of the first substrate and the second substrate comprises a distal edge extending along a portion of the waist opening; and a panel layer extending longitudinally from a first lateral edge to a second lateral edge, the panel layer connected with at least one of the first substrate and the second substrate; and wherein the first substrate is bonded directly with the second substrate with a first plurality of discrete bonds arranged in a first pattern; and wherein the panel layer is bonded directly with the first substrate or the second substrate with a second plurality of discrete bonds arranged in a second pattern, wherein the first pattern is the different from the second pattern.

An absorbent article comprising: a chassis comprising a topsheet, a backsheet, and an absorbent core positioned between the topsheet and the backsheet, the chassis further comprising a first end region and a second end region longitudinally separated from the first end region by a crotch region; a first belt connected with the first end region of the chassis; a second belt connected with the second end region of the chassis, wherein laterally opposing end portions of the second belt are connected with laterally opposing end portions of the first belt to form a waist opening; wherein at least one of the first belt and the second belt comprises: a distal edge extending along a portion of the waist opening; an elastic material positioned between and connected with a first substrate and a second substrate, the elastic material comprising a first plurality of elastic strands positioned between and connected with a first substrate and a second substrate, wherein the first plurality of elastic strands comprises an Average-Strand-Spacing from about 0.25 mm to about 4 mm and an Average-Dtex from about 10 to about 500; wherein the first substrate and the second substrate each comprise a wearer facing surface, a garment facing surface; and a panel layer connected with the wearer surface of the second substrate, the panel layer comprising a first lateral edge and a second lateral edge, wherein the first lateral edge is positioned longitudinally outboard of the second lateral edge.

An absorbent article comprising: a chassis comprising a topsheet, a backsheet, and an absorbent core positioned between the topsheet and the backsheet, the chassis further comprising a first end region and a second end region longitudinally separated from the first end region by a crotch region; a first belt connected with the first end region of the chassis; a second belt connected with the second end region of the chassis, wherein laterally opposing end portions of the second belt are connected with laterally opposing end portions of the first belt to form a waist opening; wherein at least one of the first belt and the second belt comprises: a first plurality of elastic strands positioned between and connected with a first substrate and a second substrate, wherein the first plurality of elastic strands comprises an Average-Strand-Spacing from about 0.25 mm to about 4 mm and an Average-Dtex from about 10 to about 500; and wherein the first substrate and the second substrate each comprise a wearer facing surface, a garment facing surface; and wherein the first substrate comprises first apertures.

An absorbent article comprising: a chassis comprising a topsheet, a backsheet, and an absorbent core positioned between the topsheet and the backsheet, the chassis further comprising a first end region and a second end region longitudinally separated from the first end region by a crotch region; a first belt comprising a first end region laterally separated from a second end region by a central region, the central region connected with the first end region of the chassis; a second belt comprising a first end region laterally separated from a second end region by a central region, the central region connected with the second end region of the chassis, wherein first and second end regions of the second belt are connected with respective first and second end regions of the first belt to form a waist opening; wherein at least one of the first belt and the second belt comprises: an elastic material positioned between and connected with a first substrate and a second substrate; wherein the first substrate and the second substrate each comprise a wearer facing surface, a garment facing surface; and wherein at least one of the first substrate and the second substrate comprises a distal edge extending along a portion of the waist opening and a proximal edge extending across the backsheet.

BRIEF DESCRIPTION OF THE DRAWINGS

Figure 1 shows a perspective views of a diaper pant in a pre-fastened configuration.

Figure 2A shows a plan view of a diaper pant with the portion of the diaper that faces away from a wearer oriented toward the viewer.

Figure 2B shows a plan view of a diaper pant with the portion of the diaper that faces toward a wearer oriented toward the viewer.

Figure 2C shows a plan view of a diaper pant with the portion of the diaper that faces away from a wearer oriented toward the viewer, illustrating first and second belt size and shape features.

Figure 2D shows a plan view of a diaper pant with the portion of the diaper that faces away from a wearer oriented toward the viewer, illustrating first and second belt size and shape features.

Figure 2E shows a plan view of a diaper pant with the portion of the diaper that faces away from a wearer oriented toward the viewer, illustrating first and second belt size and shape features.

Figure 3 is a cross-sectional view of the diaper pant of Figure 2A taken along line 3-3 showing first and second elastic belts provided with panel layers.

Figure 3A is a partial cross-sectional view of an alternate configuration of a panel layer.

Figure 3B is a partial cross-sectional view of a panel layer connected with a wearer facing surface of an elastic belt substrate.

Figure 3C is a partial cross-sectional view of a panel layer connected with a wearer facing surface of an elastic belt substrate and partially overlapping an end region of a chassis.

Figure 4A is a partial cross-sectional view of a panel layer configured as a laminate connected with a garment facing surface of an elastic belt substrate.

Figure 4B is a partial cross-sectional view of a panel layer configured as a laminate connected with a wearer facing surface of an elastic belt substrate.

Figure 4C is a partial cross-section view of a panel layer configured as a laminate defined by a folded substrate.

Figure 4D is a partial cross-sectional view of a panel layer configured as a laminate defined by a folded substrate connected with a wearer facing surface of an elastic belt substrate.

Figure 4E is a partial cross-sectional view of a panel layer configured as a laminate defined by a folded substrate connected with a wearer facing surface and a garment facing surface of elastic belt substrates.

Figure 4F is a partial cross-sectional view of a panel layer configured as a laminate defined by at least two substrates connected with a wearer facing surface and a garment facing surface of elastic belt substrates.

Figure 5A is a cross-sectional view of the diaper pant of Figure 2A taken along line 3-3 including first and second elastic belts with second substrates configured as laminates.

Figure 5B is a cross-sectional view of the diaper pant of Figure 2A taken along line 3-3 including first and second elastic belts with first substrates configured as laminates.

Figure 5C is a cross-sectional view of a diaper pant including first and second elastic belts with first substrates configured as laminates and connecting the first and second elastic belts.

Figure 5D is a cross-sectional view of a diaper pant including first and second elastic belts with a first substrate connecting the first and second elastic belts.

Figure 6A is a partial cross-sectional view of an elastic belt including a folded portion of a substrate and connected with a garment facing surface of the elastic belt.

Figure 6B is a partial cross-sectional view of an elastic belt including a folded portion of a substrate and connected with a wearer facing surface of the elastic belt.

Figure 6C is a partial cross-sectional view of an elastic belt including a folded portion connected with a wearer facing surface of the elastic belt.

Figure 6D is a partial cross-sectional view of an elastic belt including a folded portion connected with a wearer facing surface of the elastic belt and overlapping an end portion of a chassis.

Figure 7 shows a perspective views of a diaper pant in a pre-fastened configuration illustrating bonds and bond patterns on first substrates, second substrates, and panel layers on first and second elastic belts.

Figure 8 shows a perspective views of a diaper pant in a pre-fastened configuration illustrating apertures on first substrates, second substrates, and panel layers on first and second elastic belts.

Figure 9 shows a perspective views of a diaper pant in a pre-fastened configuration illustrating rugosities on first substrates, second substrates, and panel layers on first and second elastic belts.

## DETAILED DESCRIPTION OF THE INVENTION

## DEFINITIONS

The following term explanations may be useful in understanding the present disclosure:

"Absorbent article" refers to devices, which absorb and contain body exudates and, more specifically, refers to devices, which are placed against or in proximity to the body of the wearer to absorb and contain the various exudates discharged from the body. Exemplary absorbent articles include diapers, training pants, pull-on pant-type diapers (i.e., a diaper having a pre-formed waist opening and leg openings such as illustrated in U.S. Pat. No. 6,120,487), refastenable diapers or pant-type diapers, incontinence briefs and undergarments, diaper holders and liners, feminine hygiene garments such as panty liners, absorbent inserts, menstrual pads and the like.

"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).

An "elastic," "elastomer" or "elastomeric" refers to materials exhibiting elastic properties, which include any material that upon application of a force to its relaxed, initial length can stretch or elongate to an elongated length more than 10% greater than its initial length and will substantially recover back to about its initial length upon release of the applied force. Elastomeric materials may include elastomeric films, scrims, nonwovens, ribbons, strands and other sheet-like structures.

As used herein, the term "joined" encompasses configurations whereby an element is directly secured to another element by affixing the element directly to the other element, and configurations whereby an element is indirectly secured to another element by affixing the element to intermediate member(s) which in turn are affixed to the other element.

As used herein, the term "distal" is used to describe a position situated away from a center of a body or from a point of attachment, and the term "proximal" is used to describe a position situated nearer to a center of a body or a point of attachment.

The term "substrate" is used herein to describe a material which is primarily two-dimensional (i.e., in an XY plane) and whose thickness (in a Z direction) is relatively small (i.e., 1/10 or less) in comparison to its length (in an X direction) and width (in a Y direction). Nonlimiting examples of substrates include a web, layer or layers or fibrous materials, nonwovens, films and foils such as polymeric films or metallic foils. These materials may be used alone or may comprise two or more layers laminated together. As such, a web is a substrate.

The term "nonwoven" refers herein to a material made from continuous (long) filaments (fibers) and/or discontinuous (short) filaments (fibers) by processes such as spunbonding, meltblowing, carding, and the like. Nonwovens do not have a woven or knitted filament pattern.

The term "machine direction" (MD) is used herein to refer to the direction of material flow through a process. In addition, relative placement and movement of material can be described as flowing in the machine direction through a process from upstream in the process to downstream in the process.

The term "cross direction" (CD) is used herein to refer to a direction that is generally perpendicular to the machine direction.

"Pre-strain" refers to the strain imposed on an elastic or elastomeric material prior to combining it with another element of the elastomeric laminate or the absorbent article. Pre-strain is determined by the following equation Pre-strain = ((extended length of the elastic-relaxed length of the elastic)/relaxed length of the elastic)*100.

"Decitex" also known as Dtex is a measurement used in the textile industry used for measuring yarns or filaments. 1 Decitex = 1 gram per 10,000 meters. In other words, if 10,000 linear meters of a yarn or filament weights 500 grams that yarn or filament would have a decitex of 500.

The term "taped diaper" (also referred to as "open diaper") refers to disposable absorbent articles having an initial front waist region and an initial back waist region that are not fastened, pre-fastened, or connected to each other as packaged, prior to being applied to the wearer. A taped diaper may be folded about the lateral centerline with the interior of one waist region in surface to surface contact with the interior of the opposing waist region without fastening or joining the waist regions together. Example taped diapers are disclosed in various suitable configurations U.S. Patent Nos. 5,167,897, 5,360,420, 5,599,335, 5,643,588, 5,674,216, 5,702,551, 5,968,025, 6,107,537, 6,118,041, 6,153,209, 6,410,129, 6,426,444, 6,586,652, 6,627,787, 6,617,016, 6,825,393, and 6,861,571; and U.S. Patent Publication Nos. 2013/0072887 A1; 2013/0211356 A1; and 2013/0306226 A1.

The term "pant" (also referred to as "training pant", "pre-closed diaper", "diaper pant", "pant diaper", and "pull-on diaper") refers herein to disposable absorbent articles having a continuous perimeter waist opening and continuous perimeter leg openings designed for infant or adult wearers. A pant can be configured with a continuous or closed waist opening and at least one continuous, closed, leg opening prior to the article being applied to the wearer. A pant can be preformed or pre-fastened by various techniques including, but not limited to, joining together portions of the article using any refastenable and/or permanent closure member (e.g., seams, heat bonds, pressure welds, adhesives, cohesive bonds, mechanical fasteners, etc.). A pant can be preformed anywhere along the circumference of the article in the waist region (e.g., side fastened or seamed, front waist fastened or seamed, rear waist fastened or seamed). Example diaper pants in various configurations are disclosed in U.S. Patent Nos. 4,940,464; 5,092,861; 5,246,433; 5,569,234; 5,897,545; 5,957,908; 6,120,487; 6,120,489; 7,569,039 and U.S. Patent Publication Nos. 2003/0233082 A1; 2005/0107764 A1, 2012/0061016 A1, 2012/0061015 A1; 2013/0255861 A1; 2013/0255862 A1; 2013/0255863 A1; 2013/0255864 A1; and 2013/0255865 A1.

"Closed-form" means opposing waist regions are joined, as packaged, either permanently or refastenably to form a continuous waist opening and leg openings.

"Open-form" means opposing waist regions are not initially joined to form a continuous waist opening and leg openings but comprise a closure means such as a fastening system to join the waist regions to form the waist and leg openings before or during application to a wearer of the article.

The present disclosure relates to absorbent articles including belts, and more particularly, to front and back waist belt assemblies. In some configurations, an absorbent article may include a chassis comprising a topsheet, a backsheet, and an absorbent core positioned between the topsheet and the backsheet. The chassis may further comprise a first end region and a second end region longitudinally separated from the first end region by a crotch region. A first belt may be connected with the first end region of the chassis; and a second belt may be connected with the second end region of the chassis, wherein laterally opposing end portions of the second belt are connected with laterally opposing end portions of the first belt to form a waist opening. At least one of the first belt and the second belt may comprise: an elastic material positioned between and connected with a first substrate and a second substrate. A panel layer extending longitudinally from a first lateral edge to a second lateral edge may be connected with at least one of the first substrate and the second substrate. As discussed below, the panel layer and/or the first and/or second substrate of the first and/or second belts may include features that may differ along a width and/or length of the belts. Such features may include, for example, belt tensioning, rugosities, bonding patterns, and aperture arrangements.

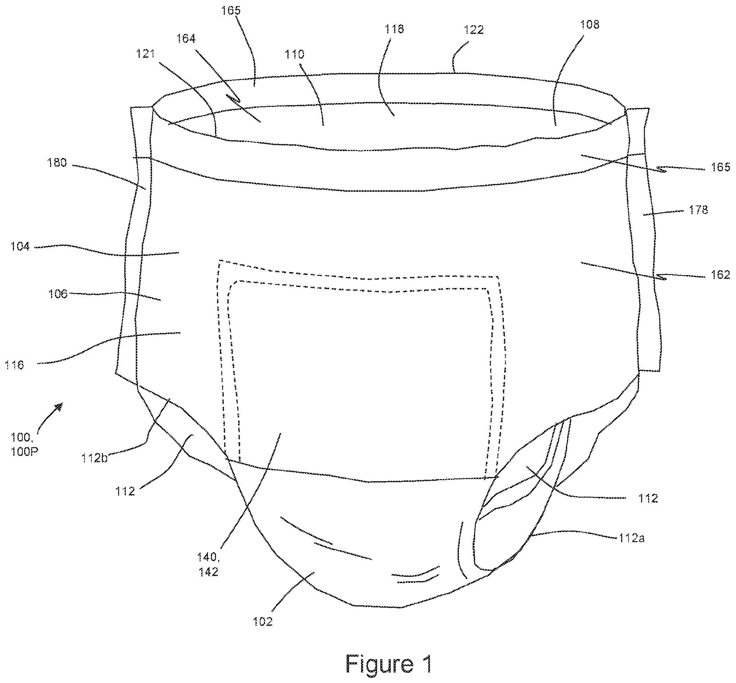

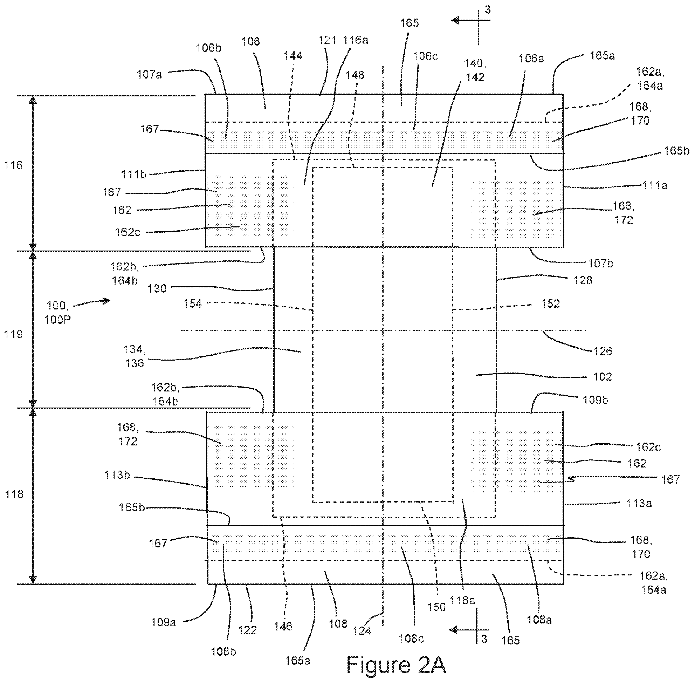

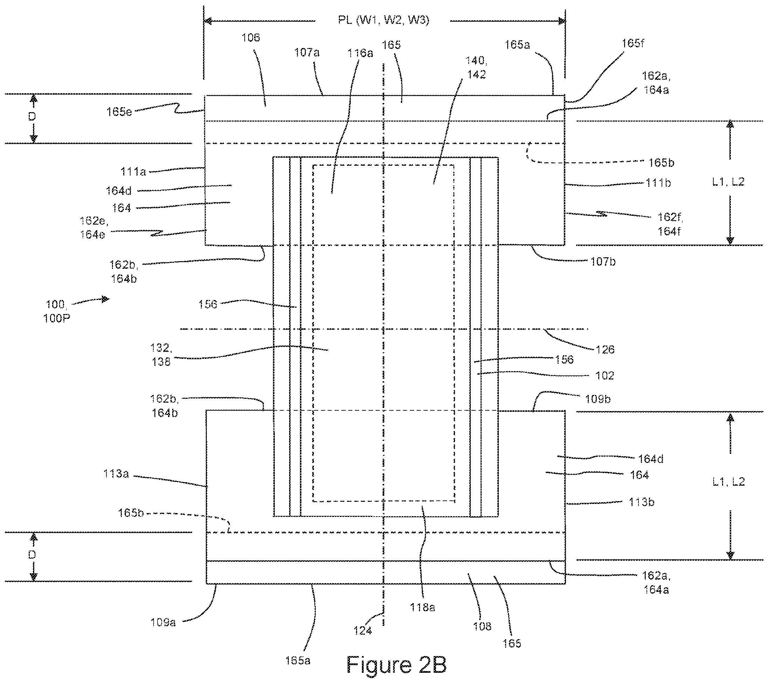

Figures 1-2B show an example of an absorbent article 100 in the form of a diaper pant 100P that may include components constructed from elastomeric laminates assembled in accordance with the configurations disclosed herein. In particular, Figure 1 shows a perspective views of a diaper pant 100P in a pre-fastened configuration. Figure 2A shows a plan view of the diaper pant 100P with the portion of the diaper that faces away from a wearer oriented toward the viewer, and Figure 2B shows a plan view of the diaper pant 100P with the portion of the diaper that faces toward a wearer oriented toward the viewer. The diaper pant 100P includes a chassis 102 and a ring-like elastic belt 104. As discussed below in more detail, a first elastic belt 106 and a second elastic belt 108 are bonded together to form the ring-like elastic belt 104.

With continued reference to Figures 1-2B, the diaper pant 100P and the chassis 102 each include a first waist region 116, a second waist region 118, and a crotch region 119 disposed intermediate the first and second waist regions. It may also be described that the chassis 102 includes a first end region 116a, a second end region 118a, and a crotch region 119 disposed intermediate the first and second end regions 116a, 118a. The first waist region 116 may be configured as a front waist region, and the second waist region 118 may be configured as back waist region. The diaper 100P may also include a laterally extending front waist edge 121 in the front waist region 116 and a longitudinally opposing and laterally extending back waist edge 122 in the back waist region 118. To provide a frame of reference for the present discussion, the diaper 100P and chassis 102 of Figures 2A and 2B are shown with a longitudinal axis 124 and a lateral axis 126. In some embodiments, the longitudinal axis 124 may extend through the front waist edge 121 and through the back waist edge 122. And the lateral axis 126 may extend through a first longitudinal or right side edge 128 and through a second longitudinal or left side edge 130 of the chassis 102.

As shown in Figures 1-2B, the diaper pant 100P may include an inner, body facing surface 132, and an outer, garment facing surface 134. The chassis 102 may include a backsheet 136 and a topsheet 138. The chassis 102 may also include an absorbent assembly 140, including an absorbent core 142, disposed between a portion of the topsheet 138 and the backsheet 136. As discussed in more detail below, the diaper 100P may also include other features, such as leg elastics and/or leg cuffs to enhance the fit around the legs of the wearer.

As shown in Figure 2A, the periphery of the chassis 102 may be defined by the first longitudinal side edge 128, a second longitudinal side edge 130, a first laterally extending end edge 144 disposed in the first waist region 116, and a second laterally extending end edge 146 disposed in the second waist region 118. Both side edges 128 and 130 extend longitudinally between the first end edge 144 and the second end edge 146. As shown in Figure 2A, the laterally extending end edges 144 and 146 are located longitudinally inward from the laterally extending front waist edge 121 in the front waist region 116 and the laterally extending back waist edge 122 in the back waist region 118. When the diaper pant 100P is worn on the lower torso of a wearer, the front waist edge 121 and the back waist edge 122 may encircle a portion of the waist of the wearer. At the same time, the side edges 128 and 130 may encircle at least a portion of the legs of the wearer. And the crotch region 119 may be generally positioned between the legs of the wearer with the absorbent core 142 extending from the front waist region 116 through the crotch region 119 to the back waist region 118.

As previously mentioned, the diaper pant 100P may include a backsheet 136. The backsheet 136 may also define the outer, garment facing surface 134 of the chassis 102. The backsheet 136 may also comprise a woven or nonwoven material, polymeric films such as thermoplastic films of polyethylene or polypropylene, and/or a multi-layer or composite materials comprising a film and a nonwoven material. The backsheet may also comprise an elastomeric film. An example backsheet 136 may be a polyethylene film having a thickness of from about 0.012 mm (0.5 mils) to about 0.051 mm (2.0 mils). Further, the backsheet 136 may permit vapors to escape from the absorbent core (i.e., the backsheet is breathable) while still preventing exudates from passing through the backsheet 136.

Also described above, the diaper pant 100P may include a topsheet 138. The topsheet 138 may also define all or part of the inner, wearer facing surface 132 of the chassis 102. The topsheet 138 may be liquid pervious, permitting liquids (e.g., menses, urine, and/or runny feces) to penetrate through its thickness. A topsheet 138 may be manufactured from a wide range of materials such as woven and nonwoven materials; apertured or hydroformed thermoplastic films; apertured nonwovens, porous foams; reticulated foams; reticulated thermoplastic films; and thermoplastic scrims. Woven and nonwoven materials may comprise natural fibers such as wood or cotton fibers; synthetic fibers such as polyester, polypropylene, or polyethylene fibers; or combinations thereof. If the topsheet 138 includes fibers, the fibers may be spunbond, carded, wet-laid, meltblown, hydroentangled, or otherwise processed as is known in the art. Topsheets 138 may be selected from high loft nonwoven topsheets, apertured film topsheets and apertured nonwoven topsheets. Exemplary apertured films may include those described in U.S. Patent Nos. 5,628,097; 5,916,661; 6,545,197; and 6,107,539.

As mentioned above, the diaper pant 100P may also include an absorbent assembly 140 that is joined to the chassis 102. As shown in Figure 2A, the absorbent assembly 140 may have a laterally extending front edge 148 in the front waist region 116 and may have a longitudinally opposing and laterally extending back edge 150 in the back waist region 118. The absorbent assembly may have a longitudinally extending right side edge 152 and may have a laterally opposing and longitudinally extending left side edge 154, both absorbent assembly side edges 152 and 154 may extend longitudinally between the front edge 148 and the back edge 150. The absorbent assembly 140 may additionally include one or more absorbent cores 142 or absorbent core layers. The absorbent core 142 may be at least partially disposed between the topsheet 138 and the backsheet 136 and may be formed in various sizes and shapes that are compatible with the diaper. Exemplary absorbent structures for use as the absorbent core of the present disclosure are described in U.S. Patent Nos. 4,610,678; 4,673,402; 4,888,231; and 4,834,735.

Some absorbent core embodiments may comprise fluid storage cores that contain reduced amounts of cellulosic airfelt material. For instance, such cores may comprise less than about 40%, 30%, 20%, 10%, 5%, or even 1% of cellulosic airfelt material. Such a core may comprise primarily absorbent gelling material in amounts of at least about 60%, 70%, 80%, 85%, 90%, 95%, or even about 100%, where the remainder of the core comprises a microfiber glue (if applicable). Such cores, microfiber glues, and absorbent gelling materials are described in U.S. Patent Nos. 5,599,335; 5,562,646; 5,669,894; and 6,790,798 as well as U.S. Patent Publication Nos. 2004/0158212 A1 and 2004/0097895 A1.

As previously mentioned, the diaper 100P may also include elasticized leg cuffs 156. It is to be appreciated that the leg cuffs 156 can be and are sometimes also referred to as leg bands, side flaps, barrier cuffs, elastic cuffs or gasketing cuffs. The elasticized leg cuffs 156 may be configured in various ways to help reduce the leakage of body exudates in the leg regions. Example leg cuffs 156 may include those described in U.S. Patent Nos. 3,860,003; 4,909,803; 4,695,278; 4,795,454; 4,704,115; 4,909,803; and U.S. Patent Publication No. 2009/0312730 A1.

As mentioned above, diaper pants may be manufactured with a ring-like elastic belt 104 and provided to consumers in a configuration wherein the front waist region 116 and the back waist region 118 are connected to each other as packaged, prior to being applied to the wearer. As such, diaper pants may have a continuous perimeter waist opening 110 and continuous perimeter leg openings 112 such as shown in Figure 1. The ring-like elastic belt may be formed by joining a first elastic belt to a second elastic belt with a permanent side seam or with an openable and reclosable fastening system disposed at or adjacent the laterally opposing sides of the belts.

As previously mentioned, the ring-like elastic belt 104 may be defined by a first elastic belt 106 connected with a second elastic belt 108. As shown in Figures 2A and 2B, the first elastic belt 106 extends between a first longitudinal side edge 111a and a second longitudinal side edge 111b and defines first and second opposing end regions 106a, 106b and a central region 106c. And the second elastic 108 belt extends between a first longitudinal side edge 113a and a second longitudinal side edge 113b and defines first and second opposing end regions 108a, 108b and a central region 108c. The distance between the first longitudinal side edge 111a and the second longitudinal side edge 111b defines the pitch length, PL, of the first elastic belt 106, and the distance between the first longitudinal side edge 113a and the second longitudinal side edge 113b defines the pitch length, PL, of the second elastic belt 108. The central region 106c of the first elastic belt is connected with the first waist region 116 or first end region 116a of the chassis 102, and the central region 108c of the second elastic belt 108 is connected with the second waist region 118 or second end region 118a of the chassis 102. As shown in Figure 1, the first end region 106a of the first elastic belt 106 is connected with the first end region 108a of the second elastic belt 108 at first side seam 178, and the second end region 106b of the first elastic belt 106 is connected with the second end region 108b of the second elastic belt 108 at second side seam 180 to define the ring-like elastic belt 104 as well as the waist opening 110 and leg openings 112. It is to be appreciated that the first belt 106 and the second belt 108 may be permanently or refastenably connected with each other at the first side seam 178 and the second side seam 180.

As shown in Figures 2A and 2B, the first elastic belt 106 also defines an outer laterally extending edge 107a and an inner laterally extending edge 107b, and the second elastic belt 108 defines an outer laterally extending edge 109a and an inner laterally extending edge 109b. As such, a perimeter edge 112a of one leg opening may be defined by portions of the inner laterally extending edge 107b of the first elastic belt 106, the inner laterally extending edge 109b of the second elastic belt 108, and the first longitudinal or right side edge 128 of the chassis 102. And a perimeter edge 112b of the other leg opening may be defined by portions of the inner laterally extending edge 107b, the inner laterally extending edge 109b, and the second longitudinal or left side edge 130 of the chassis 102. The outer laterally extending edges 107a, 109a may also define the front waist edge 121 and the laterally extending back waist edge 122 of the diaper pant 100P.

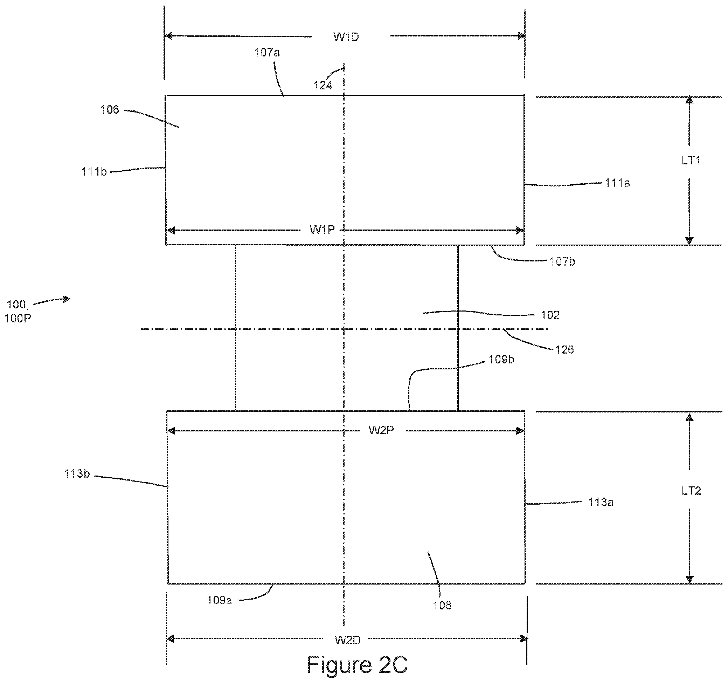

It is to be appreciated that the first elastic belt 106 and the second elastic belt 108 may define different sizes and shapes. For example, Figure 2C shows a configuration wherein the first elastic belt 106 and the second elastic belt 108 both define generally rectangular shapes. For example, as shown in Figure 2C, the outer laterally extending edge 107a of the first elastic belt 106 may comprise a lateral width of W1D and the inner laterally extending edge 107b may comprise a lateral width of W1P, wherein W1D and W1P are equal or substantially equal. In addition, the outer laterally extending edge 109a of the second elastic belt 108 may comprise a lateral width of W2D and the inner laterally extending edge 109b may comprise a lateral width of W2P, wherein W2D and W2P are equal or substantially equal.

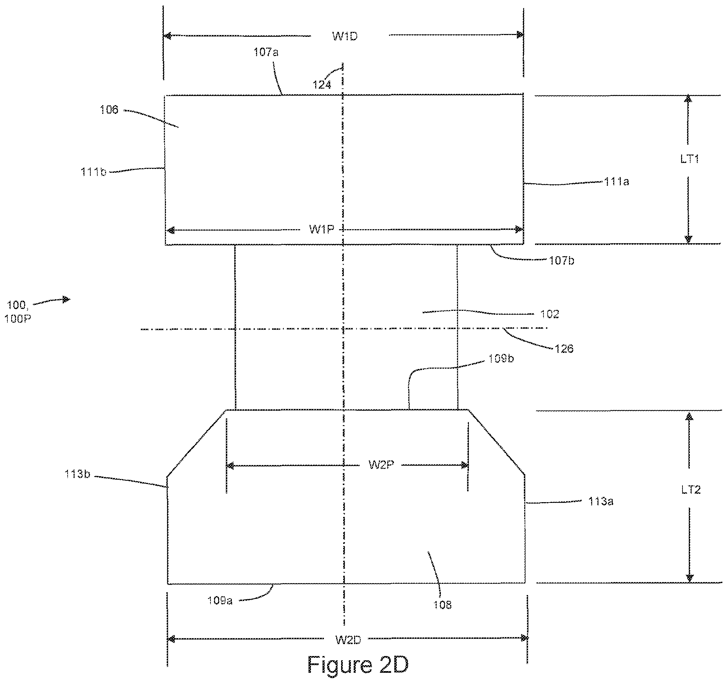

In some configurations, at least one of the first elastic belt 106 and the second elastic belt 108 may comprise lateral edges having different lengths. For example, Figure 2D shows a configuration wherein the first elastic belt 106 defines a generally rectangular shape, such as described with reference to Figure 2C, and wherein the outer laterally extending edge 109a of the second elastic belt 108 and the inner laterally extending edge 109b have different lengths. As shown in Figure 2D, the outer laterally extending edge 109a of the second elastic belt 108 may comprise a lateral width of W2D and the inner laterally extending edge 109b may comprise a lateral width of W2P, wherein W2D is greater than W2P.

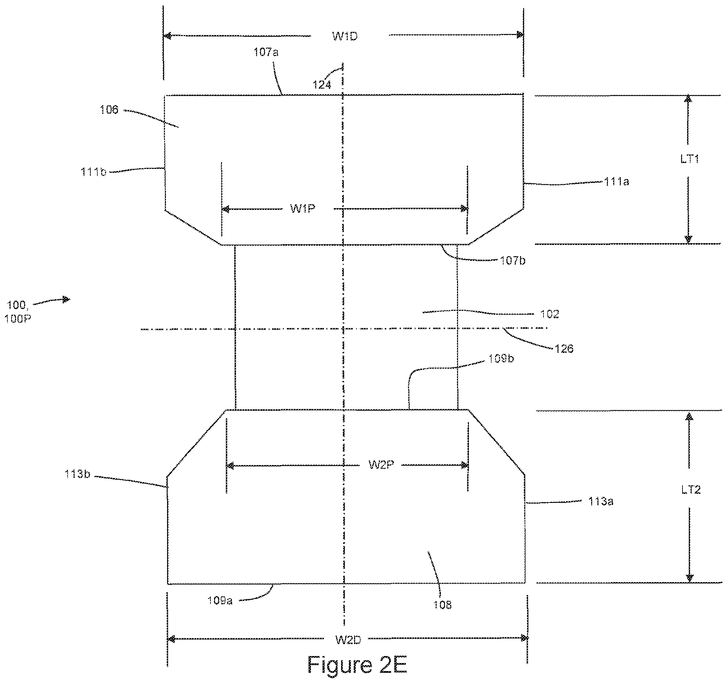

In some configurations, both the first elastic belt 106 and the second elastic belt 108 may comprise lateral edges having different lengths. For example, Figure 2E shows a configuration wherein the outer laterally extending edge 107a of the first elastic belt 106 and the inner laterally extending edge 107b have different lengths, and wherein the outer laterally extending edge 109a of the second elastic belt 108 and the inner laterally extending edge 109b have different lengths. As shown in Figure 2E, the outer laterally extending edge 107a of the first elastic belt 107 may comprise a lateral width of W1D and the inner laterally extending edge 107b may comprise a lateral width of W1P, wherein W1D is greater than W1P, and wherein the outer laterally extending edge 109a of the second elastic belt 108 may comprise a lateral width of W2D and the inner laterally extending edge 109b may comprise a lateral width of W2P, wherein W2D is greater than W2P.

With reference to Figures 2C-2E, the first elastic belt 106 may define a longitudinal length LT1 extending between outer laterally extending edge 107a and the inner laterally extending edge 107b, and the second elastic belt 108 may define a longitudinal length LT2 extending between outer laterally extending edge 109a and the inner laterally extending edge 109b. In some configurations, LT1 may be equal to LT2. In some configurations, LT1 may be less or greater than LT2. With continued reference to Figures 2C-2E, in some configurations, W1D may be equal to W1P, or W1D may be different than W1P. In some configurations, W2D may be equal to W2P, or W2D may be different than W2P. In some configurations, W1D and/or W1P may be equal to or different W2D and/or W2P.

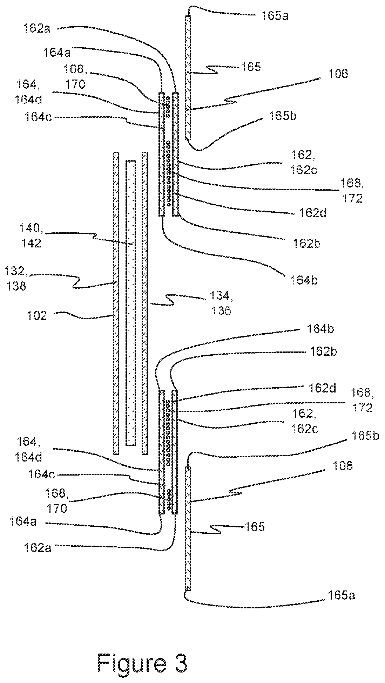

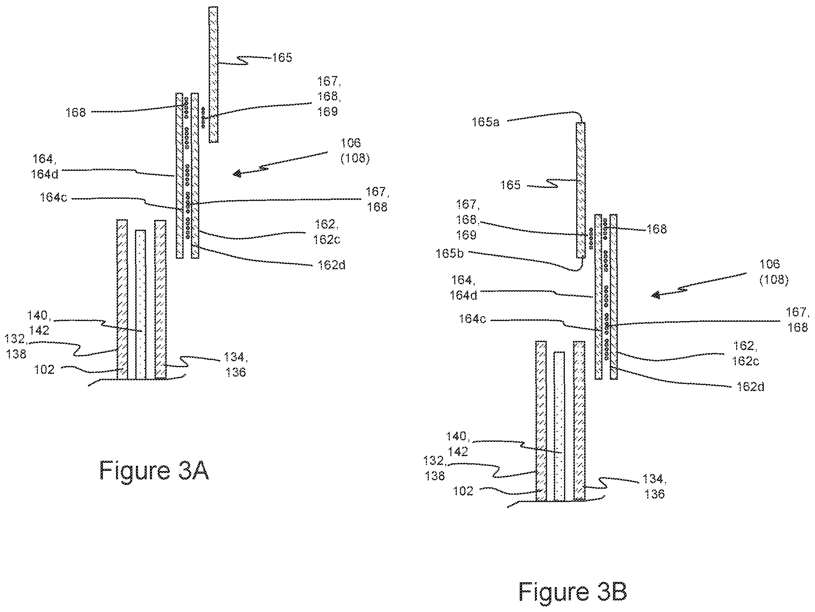

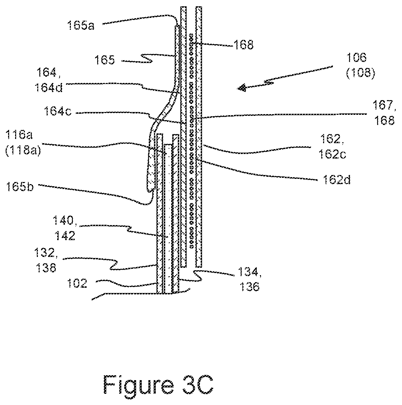

With reference to Figures 2A, 2B, and 3, the first elastic belt 106 and the second elastic belt 108 may also each include a first substrate 162 and a second substrate 164. The first substrates 162 may be oriented to define at least a portion of the garment facing surfaces of the first elastic belt 106 and the second elastic belt 108, and the second substrates 164 may be oriented to define at least a portion of the wearer facing surfaces of the first elastic belt 106 and the second elastic belt 108. The first substrate 162 may extend from a proximal edge 162b to a distal edge 162a for a maximum length L1, and the second substrate 164 may extend from a proximal edge 164b to a distal edge 164a for a maximum length L2. It is to be appreciated that the distal edge 162a and/or the proximal edge 162b of the first substrate 162 may be straight and/or curved and/or may be parallel or unparallel to each other. It is also to be appreciated that the distal edge 164a and/or the proximal edge 164b of the second substrate 164 may be straight and/or curved and/or may be parallel or unparallel to each other. As such, the maximum length L1 refers to the longest distance extending longitudinally between the distal edge 162a and the proximal edge 162b of the first substrate 162, and the maximum length L2 refers to the longest distance extending longitudinally between the distal edge 164a and the proximal edge 164b of the second substrate 164. In some configurations, the distal edge 162a of the first substrate 162 may define at least a portion of the front waist edge 121 and/or at least a portion of back waist edge 122, and/or the distal edge 164a of the second substrate 164 may define at least a portion of the front waist edge 121 and/or at least a portion of back waist edge 122. As such, in some configurations, the distal edge 162a of the first substrate 162 and/or the distal edge 164a of the second substrate 164 may define at least a portion of the waist opening 110. It is also to be appreciated that the first substrate 162 and/or the second substrate 164 may extend continuously from the first belt 106 to the second belt 108.

In some configurations, the proximal edge 162b of the first substrate 162 and/or the proximal edge 164b of the second substrate 164 may extend laterally across the backsheet 134. As shown in Figures 2A-3, the first substrate 162 includes a garment facing surface 162c and an opposing wearer facing surface 162d, and the second substrate 164 includes a garment facing surface 164c and an opposing wearer facing surface 164d.

Belt elastic material 167 may be positioned between the wearer facing surface 162d of the first substrate 162 and the garment facing surface 164c of the second substrate 164. It is to be appreciated that the belt elastic material 167 may include one or more elastic elements such as strands, ribbons, elastic films, or panels extending along the lengths of the elastic belts. As shown in Figures 2A and 3, the elastic material 167 may include a plurality of elastic strands 168. In some configurations, the elastic strands 168 may be in the form of extruded elastic strands, which may also be bonded with the first substrate 162 and/or second substrate 164 in a pre-corrugated configuration, such as disclosed for example in U.S. Patent No. 5,681,302, which is incorporated by reference herein. In some configurations, the elastic strands 168 may be referred to herein as outer, waist elastics 170 and inner, waist elastics 172. Elastic strands 168, such as the outer waist elastics 170, may continuously extend laterally between the first and second opposing end regions 106a, 106b of the first elastic belt 106 and between the first and second opposing end regions 108a, 108b of the second elastic belt 108. In some embodiments, some elastic strands 168, such as the inner waist elastics 172, may be configured with discontinuities in areas, such as for example, where the first and second elastic belts 106, 108 overlap portions of the chassis 102, such as the absorbent assembly 140.

In some configurations, the first elastic belt 106 and/or second elastic belt 108 may define curved contours. For example, the inner lateral edges 107b, 109b of the first and/or second elastic belts 106, 108 may include non-linear or curved portions in the first and second opposing end regions. Such curved contours may help define desired shapes to leg opening 112, such as for example, relatively rounded leg openings. In addition to having curved contours, the elastic belts 106, 108 may include elastic strands 168 that extend along non-linear or curved paths that may correspond with the curved contours of the inner lateral edges 107b, 109b.

As shown in Figures 2A-3, the first elastic belt 106 and/or the second elastic belt 108 may also include a panel layer 165, which may comprise a substrate or a laminate of substrate layers. The panel layer 165 may extend longitudinally between a first lateral edge 165a and a second lateral edge 165b for a maximum distance D. It is to be appreciated that the first lateral edge 165a and/or the second lateral edge 165b of the panel layer 165 may be straight and/or curved and/or may be parallel or unparallel to each other. As such, the maximum distance D refers to the longest distance extending longitudinally between the first lateral edge 165a and the second lateral edge 165b of the panel layer 165. In some configurations, the maximum distance D may or may not be equal to the maximum length L1 of the first substrate 162 and/or the maximum length L2 of the second substrate 164. In some configurations, the maximum distance D may be less than or equal to the maximum length L1 of the first substrate 162 and/or the maximum length L2 of the second substrate 164. In some configurations, D may be less than about 40% of L1 and/or L2. It is also to be appreciated that panel layer 165 may be positioned in various longitudinal positions relative to the first substrate 162 and/or the second substrate 164. For example, as shown in Figures 2A-3, the first lateral edge 165a of the panel layer 165 may be positioned longitudinally outward from the distal edge 162a of the first substrate 162 and/or the distal edge 164a of the second substrate 164. In some configurations, the first lateral edge 165a of the panel layer 165 may be positioned to be coterminous with the distal edge 162a of the first substrate 162 and/or the distal edge 164a of the second substrate 164. In some configurations, the first lateral edge 165a of the panel layer 165 may be positioned longitudinally inboard from the distal edge 162a of the first substrate 162 and/or the distal edge 164a of the second substrate 164. In some configurations, the panel layer 165 may be positioned such that the first lateral edge 165a of the panel layer 165 may define at least a portion of the front waist edge 121 and/or the back waist edge 122. As such, in some configurations, the first lateral edge 165a of the panel layer 165 may define at least a portion of the waist opening 110.

In some configurations, the panel layer 165 may be positioned so as to cover at least a portion of the first end region 116a and/or the second end region 118a of the chassis 102, such as shown in Figure 3C. As such, the second lateral edge 165b of the panel layer 165 may extend across the topsheet 138. In some configurations, the second lateral edge 165b of the panel layer 165 may extend across the backsheet 136. The second lateral edge 165b of the panel layer 165 may be longitudinally inboard or longitudinally outboard of the proximal edge 162b of the first substrate 162 and/or the proximal edge 164b of the second substrate 164. And in some configurations, the second lateral edge 165b of the panel layer 165 may be conterminous with the proximal edge 162b of the first substrate 162 and/or the proximal edge 164b of the second substrate 164. In some configurations, at least a portion of the second lateral edge 165b of the panel layer 165 may not be bonded with the wearer facing surface 164d of the second substrate 164 to define a pocket between the panel layer 165 and the wearer facing surface 164d of the second substrate 164.

It is to be appreciated that the first substrate 162, the second substrate 164, and the panel layer 165 may define various lateral widths that may or may not be equal. For example, as shown in Figure 2B, the first substrate 162 may extend laterally between a first longitudinal edge 162e and a second longitudinal edge 162f to define a first lateral width W1, and the second substrate 164 may extend laterally between a first longitudinal edge 164e and a second longitudinal edge 164f to define a second lateral width W2. In addition, the panel layer 165 may extend laterally between a first longitudinal edge 165e and second longitudinal edge 165f to define a third lateral width W3. In some configurations, the third lateral width W3 may be equal to at least one of the first lateral width W1 and the second lateral width W2. In some configurations, the third lateral width W3 may be less than at least one of the first lateral width W1 and the second lateral width W2.

It is to be appreciated that the panel layer 165 may be connected with the first elastic belt 106 and/or the second elastic belt 108 in various positions and/or with various components of the first elastic belt 106 and/or the second elastic belt 108. For example, as shown in Figure 3, the panel layer 165 may be connected with the garment facing surface 162c of the first substrate 162 on the first elastic belt 106 and/or the second elastic belt 108.

As shown in Figure 3A, the first elastic belt 106 and/or the second elastic belt 108 may include belt elastic material 167 positioned between and connected with the first substrate 162 and the panel layer 165. As mentioned above, the elastic material 167 may include a plurality of elastic strands 168. As shown in Figure 3A and others, the elastic strands 168 connected with the panel layer 165 may be referred to herein as panel layer elastic strands 169. In some configurations, the elastic strands 169 may be in the form of extruded elastic strands, which may also be bonded with the first substrate 162, second substrate 164, and/or panel layer 165 in a pre-corrugated configuration, such as disclosed for example in U.S. Patent No. 5,681,302. In another configuration, such as shown in Figure 3B, the panel layer 165 may be connected with the wearer facing surface 164d of the second substrate 164 on the first elastic belt 106 and/or the second elastic belt 108. And in some configurations, the first elastic belt 106 and/or the second elastic belt 108 may include elastic material 169 positioned between and connected with the second substrate 164 and the panel layer 165.

It is to be appreciated that the first elastic belt 106 and the second elastic belt 108 may comprise the same materials and/or may have the same structure. In some embodiments, the first elastic belt 106 and the second elastic belt may comprise different materials and/or may have different structures. It should also be appreciated that components of the first elastic belt 106 and the second elastic belt 108, such as the first substrate 162, second substrate 164, and/or panel layer 165, may be constructed from various materials. For example, the first and/or second belts may include a first substrate 162, second substrate 164, and/or panel layer 165 that may be manufactured from materials such as plastic films; apertured plastic films; woven or nonwoven webs of natural materials (e.g., wood or cotton fibers), synthetic fibers (e.g., polyolefins, polyamides, polyester, polyethylene, or polypropylene fibers) or a combination of natural and/or synthetic fibers; or coated woven or nonwoven webs. In some configurations, the first and/or second belts may include a first substrate 162, second substrate 164, and/or panel layer 165 comprising a nonwoven web of synthetic fibers, and may include a stretchable nonwoven. In some configurations, the first and second elastic belts may include an inner hydrophobic, non-stretchable nonwoven material and an outer hydrophobic, non-stretchable nonwoven material.

It is also to be appreciated that the first substrate 162, second substrate 164, panel layer 165, and/or elastic material 167 of the first elastic belt 106 and/or second elastic belt 108 may be bonded together and/or with other components, such as the chassis 102, with adhesive and/or mechanical bonds. It is to be appreciated that adhesive and mechanical bonding methods may be utilized alone or in combination with each other.

In some configurations, adhesive may be applied to at least one of the first substrate 162, second substrate 164, panel layer 165, and/or elastic material 167 when being combined to form the first elastic belt 106 and/or second elastic belt 108. Such adhesive may be applied by with adhesive applicator devices configured in various ways, such as for example, spray nozzles and/or slot coating devices. In some configurations, the adhesive applicator devices may be configured in accordance with the apparatuses and/or methods disclosed in U.S. Patent Nos. 8,186,296; 9,265,672; 9,248,054; and 9,295,590 and U.S. Patent Publication No. 2014/0148773 A1.

In some configurations, mechanical bonding devices may apply mechanical bonds to the to at least one of the first substrate 162, second substrate 164, panel layer 165, and/or elastic material 167 when being combined to form the first elastic belt 106 and/or second elastic belt 108. Such mechanical bonds may be applied with heat, pressure, and/or ultrasonic devices. Examples of ultrasonic bonding devices, which may include linear or rotary type configurations, are disclosed for example in U.S. Patent Nos. 3,113,225; 3,562,041; 3,733,238; 5,110,403; 6,036,796; 6,508,641; and 6,645,330. In some configurations, the ultrasonic bonding device may be configured as a linear oscillating type sonotrode, such as for example, available from Herrmann Ultrasonic, Inc. Examples of such mechanical bonding devices and methods are disclosed in U.S. Patent Nos. 4,854,984; 6,291,039; 6,248,195; 8,778,127; and 9,005,392; and U.S. Patent Publication Nos. 2014/0377513 A1; and 2014/0377506 A1. In some configurations, mechanical bonding devices may apply bonds that bond the first substrate 162, second substrate 164, panel layer 165, and/or elastic material 167 together and/or may act to trap or immobilize discrete lengths of the contracted elastic strands in the first elastic belt 106 and/or second elastic belt 108.

It is also to be appreciated that the first substrate 162, second substrate 164, panel layer 165, and/or elastic material 167 may be bonded together with various methods and apparatuses to create various elastomeric laminates, such as described in U.S. Patent Publication Nos. 20180168878 A1; 20180168877 A1; 20180168880 A1; 20180170027 A1; 20180169964 A1; 20180168879 A1; 20180170026 A1; 20180168889 A1; 20180168874 A1; 20180168875 A1; 20180168890 A1; 20180168887 A1; 20180168892 A1; 20180168876 A1; 20180168891 A1; 20190070042 A1; and 20190070041 A1 as well as U.S. Patent Application Nos. 62/989,059 and 62/984,837, and combinations thereof.

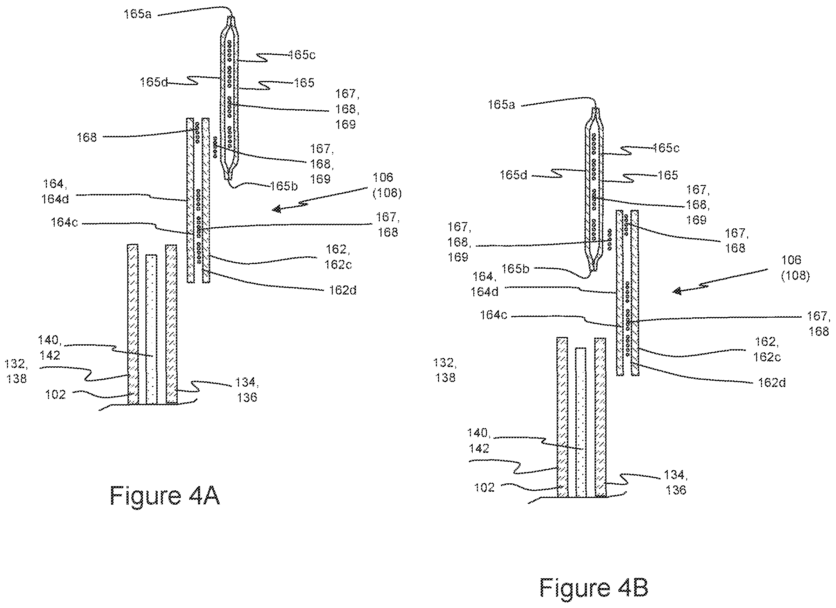

It is to be appreciated that the panel layers 165 herein may be configured as a single layer substrate or a laminate comprising two or more layers. For example, as shown in Figure 4A, the panel layer 165 may be configured as a laminate comprising a garment facing layer 165c and a wearer facing layer 165d. The panel layer 165 may also include elastic material 167, 169 positioned between and connected with the garment facing layer 165c and the wearer facing layer 165d. As previously mentioned, the panel layer 165 may be connected with the first substrate 162 or the second substrate 164 of the first elastic belt 106 and/or the second elastic belt 108. For example, as shown in Figure 4A, the wearer facing layer 165d may be connected with the garment facing surface 162c of the first substrate 162. In addition, elastic material 167, 169 may be positioned between and connected with the wearer facing layer 165d and the garment facing surface 162c of the first substrate 162. It is to be appreciated that the first lateral edge 165a of the panel layer 165 may be defined by one of or both of the garment facing layer 165c and the wearer facing layer 165d. It is also to be appreciated that the second lateral edge 165b of the panel layer 165 may be defined by one of or both of the garment facing layer 165c and the wearer facing layer 165d. In some configurations, such as shown in Figure 4B, the garment facing layer 165c may be connected with the wearer facing surface 164d of the second substrate 164. It is also to be appreciated that in some configurations, elastic material 167, 169 may be positioned between and connected with the garment facing layer 165c and the wearer facing surface 164d of the second substrate 164. It is also to be appreciated that in some configurations, elastic material 167, 169 may be positioned between and connected with the garment facing layer 165c and the wearer facing surface 164d of the second substrate 164.

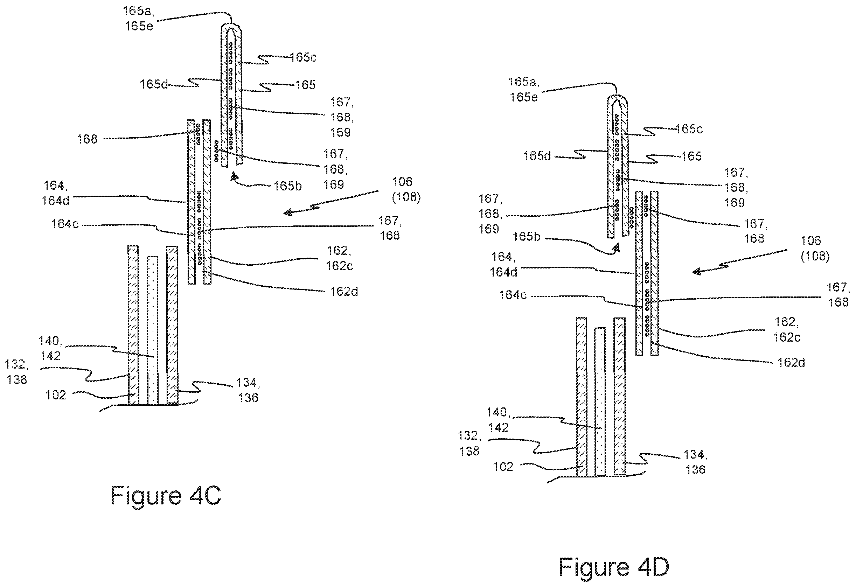

Referring now to Figure 4C, the panel layer 165 may be configured as a laminate formed by a substrate that is folded along a fold line 165e to form a first portion defining the garment facing layer 165c and a second portion defining the wearer facing layer 165d. In the folded configurations, the panel layer 165 may also include elastic material 167, 169 positioned between and connected with the garment facing layer 165c and the wearer facing layer 165d. As previously mentioned, the panel layer 165 may be connected with the first substrate 162 or the second substrate 164 of the first elastic belt 106 and/or the second elastic belt 108. For example, as shown in Figure 4C, the wearer facing layer 165d may be connected with the garment facing surface 162c of the first substrate 162. In addition, elastic material 167, 169 may be positioned between and connected with the wearer facing layer 165d and the garment facing surface 162c of the first substrate 162. It is to be appreciated that the first lateral edge 165a of the panel layer 165 may be defined by the fold line 165e. As such, in some configurations, the panel layer may be positioned such that the fold line 165e of the panel layer 165 may define at least a portion of the front waist edge 121 and/or the back waist edge 122. As such, in some configurations, the fold line 165e of the panel layer 165 may define at least a portion of the waist opening 110.

It is also to be appreciated that the second lateral edge 165b of the panel layer 165 may be defined by one of or both of the garment facing layer 165c and the wearer facing layer 165d. In some configurations, such as shown in Figure 4D, the garment facing layer 165c may be connected with the wearer facing surface 164d of the second substrate 164. It is also to be appreciated that in some configurations, elastic material 167, 169 may be positioned between and connected with the garment facing layer 165c and the wearer facing surface 164d of the second substrate 164. It is also to be appreciated that in some configurations, elastic material 167, 169 may be positioned between and connected with the garment facing layer 165c and the wearer facing surface 164d of the second substrate 164.

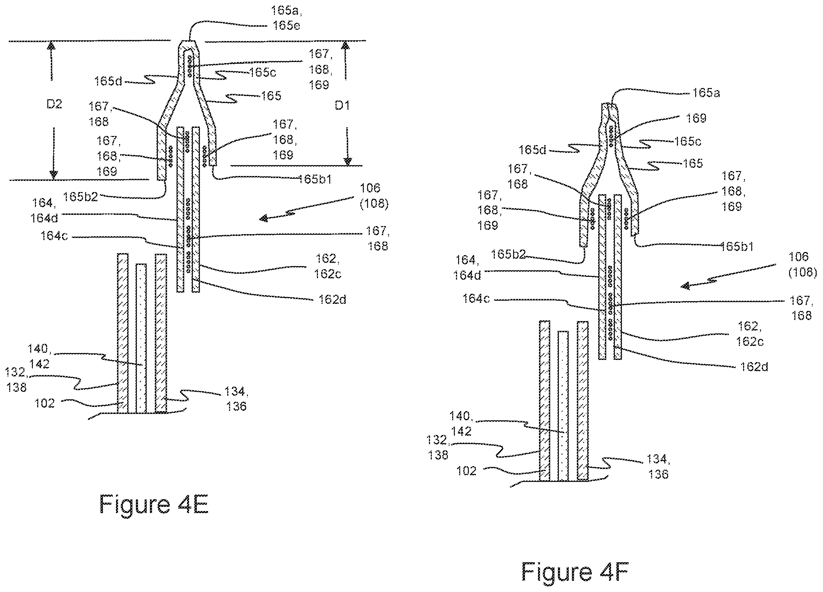

In some configurations of the first elastic belt 106 and/or the second elastic belt 108, the first substrate 162 and the second substrate 164 may be positioned between the garment facing layer 165c and the wearer facing layer 164d. More particularly, the garment facing layer 165c of the panel layer 165 may be connected with the first substrate 162 and the wearer facing layer 165d of the panel layer 165 may be connected with second substrate 164. For example, as shown in Figure 4E, the panel layer 165 is depicted as a laminate formed by a substrate that is folded along a fold line 165e to form a first portion defining the garment facing layer 165c and a second portion defining the wearer facing layer 165d. As shown in Figure 4E, the garment facing layer 165c is connected with garment facing layer 162c of the first substrate 162, and the wearer facing layer 165d is connected with wearer facing layer 164d of the second substrate 164. In addition, the panel layer 165 may also include elastic material 167, 169 positioned between and connected with the garment facing layer 165c and the wearer facing layer 165d. As shown in Figure 4E, elastic material 167, 169 may also be positioned between and connected with the garment facing layer 165c and garment facing surface 162c of the first substrate and/or positioned between and connected with the wearer facing layer 165d and the wearer facing surface 164d of the second substrate 164.

With continued reference to Figure 4E, the first lateral edge 165a of the panel layer 165 may be defined by the fold line 165e. As such, in some configurations, the panel layer may be positioned such that the fold line 165e of the panel layer 165 may define at least a portion of the front waist edge 121 and/or the back waist edge 122. As such, in some configurations, the fold line 165e of the panel layer 165 may define at least a portion of the waist opening 110. It is also to be appreciated that the garment facing layer 165c may define a first second lateral edge 165b1 of the panel layer 165 extending along the garment facing surface 162c of the first substrate 162, and the wearer facing layer 165d may define a second second lateral edge 165b2 of the panel layer 165 extending along the wearer facing surface 164d of the second substrate 164.

It is also to be appreciated that panel layer 165 may be positioned in various longitudinal positions relative to the first substrate 162 and/or the second substrate 164. For example, as shown in Figure 4E, the fold line 165e of the panel layer 165 may be positioned longitudinally outward from the distal edge 162a of the first substrate 162 and/or the distal edge 164a of the second substrate 164. In some configurations, the fold line 165e of the panel layer 165 may be positioned to extend along the distal edge 162a of the first substrate 162 and/or the distal edge 164a of the second substrate 164. In some configurations, the panel layer 165 may be positioned such that the first lateral edge 165a or fold line 165e of the panel layer 165 may define at least a portion of the front waist edge 121 and/or the back waist edge 122. As such, in some configurations, the fold line 165e of the panel layer 165 may define at least a portion of the waist opening 110.

In some configurations, the panel layer 165 may be positioned such that garment facing layer 165c and/or the wearer facing layer 165d covers at least a portion of the first end region 116 and/or the second end region 118 of the chassis 102. In some configurations, the first second lateral edge 165b1 of the panel layer 165 may extend across the backsheet 136, and/or, the second second lateral edge 165b2 of the panel layer 165 may extend across the topsheet 138. The first second lateral edge 165b1 and/or the second second lateral edge 165b2 of the panel layer 165 may be longitudinally inboard or longitudinally outboard of the proximal edge 162b of the first substrate 162 and/or the proximal edge 164b of the second substrate 164. And in some configurations, the first second lateral edge 165b1 and/or the second second lateral edge 165b2 of the panel layer 165 may be conterminous with the proximal edge 162b of the first substrate 162 and/or the proximal edge 164b of the second substrate 164. In some configurations, at least a portion of the second second lateral edge 165b2 of the panel layer 165 may not be bonded with the wearer facing surface 164d of the second substrate 164 to define a pocket between the wearer facing layer 165d of the panel layer 165 and the wearer facing surface 164d of the second substrate 164.

As shown in Figure 4E, the panel layer 165 may extend longitudinally between the first lateral edge 165a (or fold line 165e) and the first second lateral edge 165b1 for a first maximum distance D1, and the panel layer 165 may extend longitudinally between the first lateral edge 165a (or fold line 165e) and the second second lateral edge 165b2 for a second maximum distance D2. It is to be appreciated that the first lateral edge 165a and/or the first second lateral edge 165b1 of the panel layer 165 may be straight and/or curved and/or may be parallel or unparallel to each other. It is also to be appreciated that the first lateral edge 165a and/or the second second lateral edge 165b2 of the panel layer 165 may be straight and/or curved and/or may be parallel or unparallel to each other. As such, the maximum distance D1 refers to the longest distance extending longitudinally between the first lateral edge 165a and the first second edge 165b1 of the panel layer 165, and the maximum distance D2 refers to the longest distance extending longitudinally between the first lateral edge 165a and the second second edge 165b2 of the panel layer 165. In some configurations, the first maximum distance D1 and the second maximum distance D2 may be equal or different lengths. It is also to be appreciated that the first maximum distance D1 and the second maximum distance D2 may define have various lengths relative to the maximum length L1 of the first substrate 162 and the maximum length L2 of the second substrate 164 described above with reference to Figure 2B. For example, in some configurations, the first maximum distance D1 and/or the second maximum distance D2 may be less than or equal to maximum length L1 of the first substrate 162 and/or the maximum length L2 of the second substrate 164. In some configurations, the first maximum distance D1 and/or the second maximum distance D2 may be less than about 40% of L1 and/or L2. It is also to be appreciated that features of the panel layer 165 discussed above with reference to folded configuration in Figure 4E are also applicable to the panel layer 165 laminate structure that comprises at least two separate substrates bonded together, such as shown in Figure 4F.

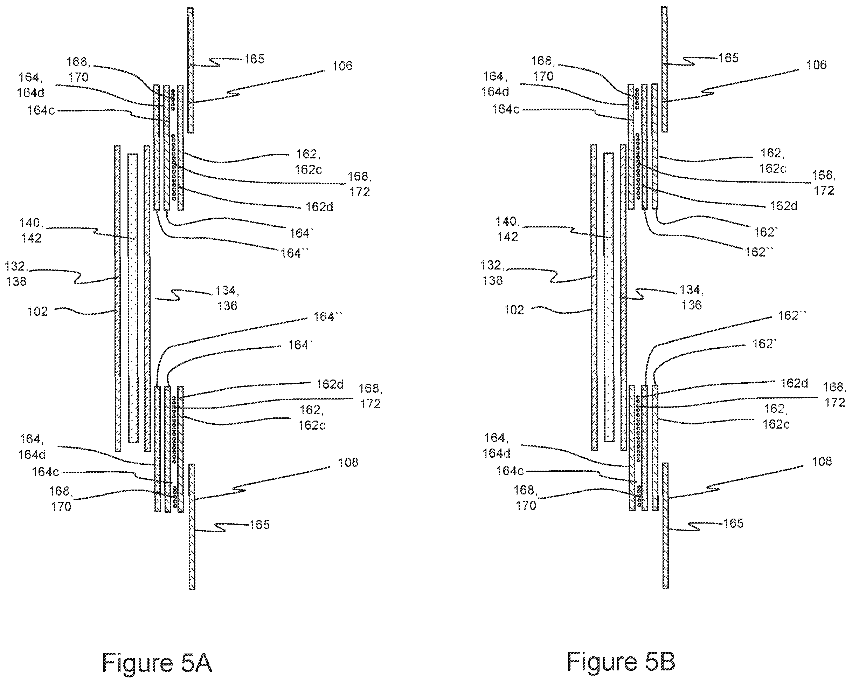

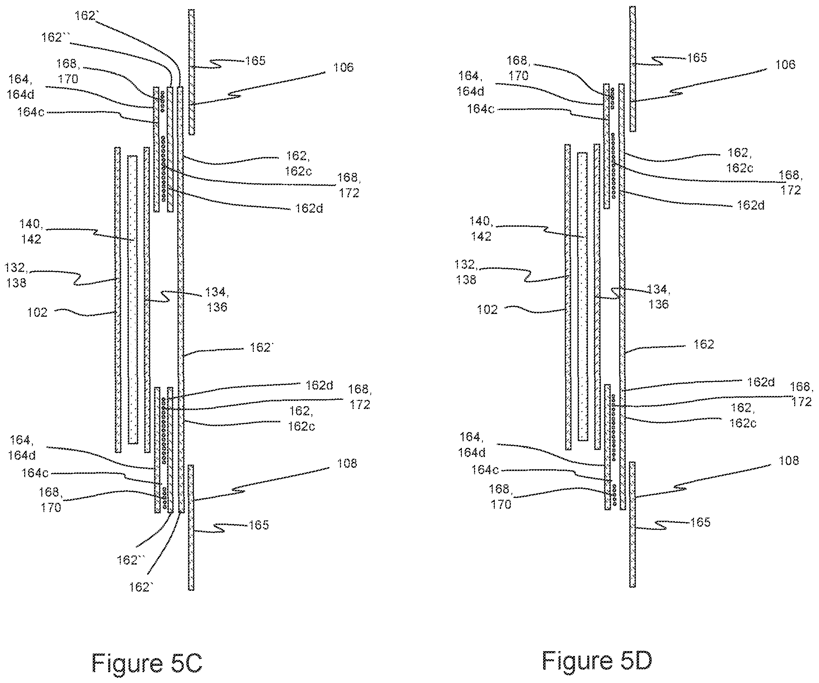

In addition to the various configurations of panel layers 165 discussed herein, it is to be appreciated that the first elastic belt 106 and the second elastic belt 108 may be configured in various ways with various configurations and/or combinations of the first substrate 162 and the second substrate 164. In some configurations, the first substrate 162 and/or the second substrate 164 may be configured as single substrate layer or a laminate structure including a plurality of substrate layers. For example, as shown in Figure 5A, the first elastic belt 106 and/or the second elastic belt 108 may include a second substrate 164 configured as a laminate comprising a garment facing layer 164' and a wearer facing layer 164". It is to be appreciated that the garment facing layer 164' and a wearer facing layer 164" may be the same or different materials. In another example, as shown in Figure 5B, the first elastic belt 106 and/or the second elastic belt 108 may include a first substrate 162 configured as a laminate comprising a garment facing layer 162' and a wearer facing layer 162". It is to be appreciated that the garment facing layer 162' and a wearer facing layer 162" may be the same or different materials. In some configurations, instead of having separate garment facing layers 162' in each of the first and second elastic belts 106, 108, a single garment facing layer 162' may be connected with and extend between the wearer facing layers 162" of the first and second elastic belts 106, 108, such as shown in Figure 5C. In yet another configuration, such as shown in Figure 5D, the first and second elastic belts 106, 108 may be configured without separate wearer facing layers 162", and as such, the first elastic belt 106 and the second elastic belt 108 may include a portion of a single garment facing layer 162' of the first substrate 162 that forms part of the first elastic belt 106 and the second elastic belt 108 and extends longitudinally between the first elastic belt 106 and the second elastic belt 108 along the backsheet 136 of the chassis 102.

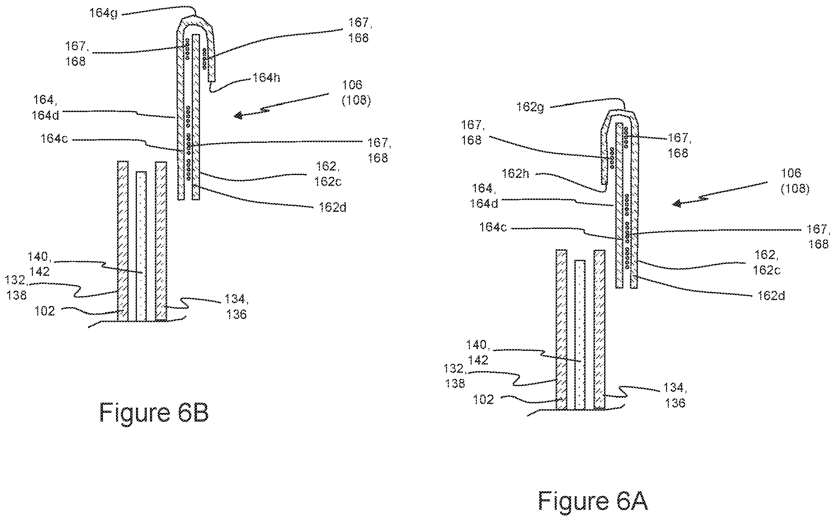

In some configurations, the first elastic belt 106 and/or the second elastic belt 108 may include a folded portion of at least the first substrate 162 and/or the second substrate 164. For example, as shown in Figure 6A, the first elastic belt 106 and/or the second elastic belt 108 may include a folded portion of the first substrate 162 extending longitudinally between a fold line 162g in the first substrate 162 and a lateral edge 162h. As such, the folded portion of the first substrate 162 may be connected with the wearer facing surface 164d of the second substrate 164. As shown in the configuration of Figure 6A, elastic material 167 may also be positioned between and connected with the folded portion of the first substrate 162 and the wearer facing surface 164d of the second substrate 164. In another example shown in Figure 6B, the first elastic belt 106 and/or the second elastic belt 108 may include a folded portion of the second substrate 164 extending longitudinally between a fold line 164g in the second substrate 164 and a lateral edge 164h. As such, the folded portion of the second substrate 164 may be connected with the garment facing surface 162c of the first substrate 162. As shown in the configuration of Figure 6B, elastic material 167 may also be positioned between and connected with the folded portion of the second substrate 164 and the garment facing surface 164c of the first substrate 162.

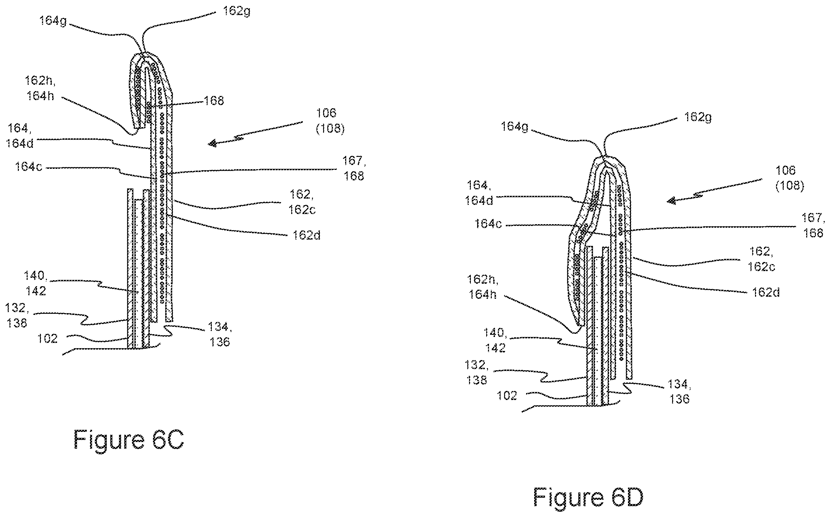

In some configurations, the first elastic belt 106 and/or the second elastic belt 108 may include a folded portion of both the first substrate 162 and the second substrate 164. For example, as shown in Figure 6C, the first elastic belt 106 and/or the second elastic belt 108 may include a folded portion of both the first substrate 162 and the second substrate 164 extending longitudinally between a fold line 162g in the first substrate 162 and a lateral edge 162h and extending longitudinally between a fold line 164g in the second substrate 164 and a lateral edge 164h. As such, the folded portion of the second substrate 164 may be connected with the wearer facing surface 164d of the second substrate 164. As shown in the configuration of Figure 6C, elastic material 167 may also be positioned between and connected with the folded portion of the second substrate 164 and the wearer facing surface 164d of the second substrate 164. It is to be appreciated the first substrate 162 and the second substrate 164 may be folded in an opposite direction than that shown in Figure 6C, such that the folded portion of the first substrate 162 may be connected with the garment facing surface 162c of the first substrate 162. In some configurations, the folded portion of the first elastic belt 106 and/or the second elastic belt 108 may configured to overlap and/or cover at least a portion of the first end region 116a and/or the second end region 118a of the chassis 102, such as shown in Figure 6D.

It is to be appreciated that components of the first elastic belt 106 and/or the second elastic belt 108 may be assembled in various ways and various combinations to create various desirable various features that may differ along the lateral width and/or longitudinal length of the first elastic belt 106 and/or the second elastic belt 108. Such features may include, for example, Dtex values, bond patterns, aperture arrangements, elastic positioning, Average Dtex values, Average Pre-Strain values, rugosity frequencies, rugosity wavelengths, height values, and/or contact area. It is to be appreciated that differing features may be imparted to various components, such as for example, the first substrate 162, second substrate 164, panel layer 165, and elastic material 167 before and/or during stages of assembly of the first elastic belt 106 and/or the second elastic belt 108. For example, differing features may be separately imparted to the panel layer 165 before being combined with the first substrate 162 and/or the second substrate 164. In another example, differing features may be separately imparted to the first substrate 162 and/or the second substrate 164 before being combined. As such, in some configurations, the first elastic belt 106 and/or the second elastic belt 108 may include differing features without the necessity to impart such features to fully assembled products.

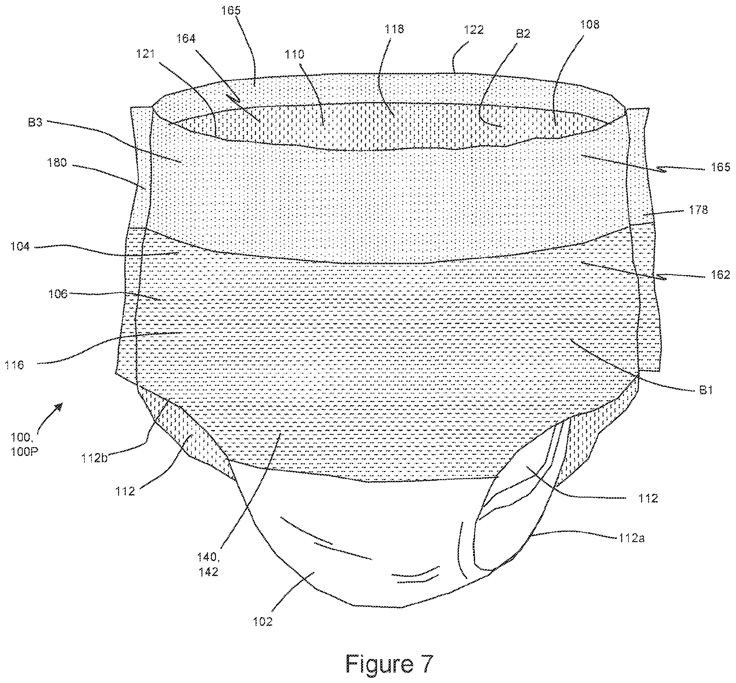

It is to be appreciated that the first substrate 162, second substrate 164, panel layer 165, and/or elastic material 167 of the first elastic belt 106 and/or second elastic belt 108 herein may be formed in various ways and may be bonded together in various ways and with differing or identical bond patterns. In some configurations, the first substrate 162, second substrate 164, panel layer 165, and/or elastic material 167 may be bonded continuously or discontinuously. In some configurations, the first substrate 162, second substrate 164, panel layer 165, and/or elastic material 167 may be bonded with a plurality of individual bond sites that may or may not form a visually discernable pattern. Such individual bond sites may also define various features with various sizes relative to each other, and such bond sites may be formed in various ways, such as with adhesive or mechanical bonds and/or combinations thereof. It is to be appreciated that the first elastic belt 106 and/or second elastic belt 108 may include various portions of components bonded together in various ways and with differing or identical bond patterns. Figure 7 shows generic examples of first bonds B1, second bonds B2, and third bonds B3 that may be arranged in first, second, and third patterns, respectively on first substrates, second substrates, and/or panel layers on the first elastic belt 106 and/or the second elastic belt 108. It is to be appreciated that the bonds may be arranged in various ways and may be arranged to bond various different components of the first elastic belt 106 and/or the second elastic belt 108.

For example, with reference Figures 1-6D, in some configurations of the first elastic belt 106 and/or second elastic belt 108, the first substrate 162 may be bonded directly with the second substrate 164 with a first plurality of discrete bonds arranged in a first pattern, and the panel layer 165 may be bonded directly with the first substrate 162 or the second substrate 164 with a second plurality of discrete bonds arranged in a second pattern, wherein the first pattern may be the same as or different from the second pattern.

In another example, the panel layer 165 may be bonded directly with the first substrate 162 with a first plurality of discrete bonds arranged in a first pattern; the panel layer 165 may be bonded directly with the second substrate 164 with a second plurality of discrete bonds arranged in a second pattern; and the first substrate 162 may be bonded directly with the second substrate 164 with a third plurality of discrete bonds arranged in a third pattern. As such, in some configurations, the first pattern may be the same or different from the second pattern. And in some configurations, at least of the first pattern and the second pattern may be different from the third pattern. Further, the first plurality of discrete bonds, the second plurality of discrete bonds, and the third plurality of discrete bonds may comprise at least one of adhesive bonds and mechanical bonds.

In yet another example, the wearer facing layer 165d of the panel layer 165 may be bonded directly with the garment facing layer 165c of the panel layer 165 with a first plurality of discrete bonds arranged in a first pattern. In addition, the wearer facing 165d of the panel layer 165 is bonded directly with the second substrate 164 and the garment facing layer 165c of the panel layer 165 is bonded directly with the first substrate 162 with a second plurality of discrete bonds arranged in a second pattern. Further, the first substrate 162 may be bonded directly with the second substrate 164 with a third plurality of discrete bonds arranged in a third pattern. In some configurations, the first pattern may be the same as the second pattern, and at least one of the first pattern and the second pattern may be different from the third pattern. In addition, the first plurality of discrete bonds, the second plurality of discrete bonds, and the third plurality of discrete bonds may comprise at least one of adhesive bonds and mechanical bonds

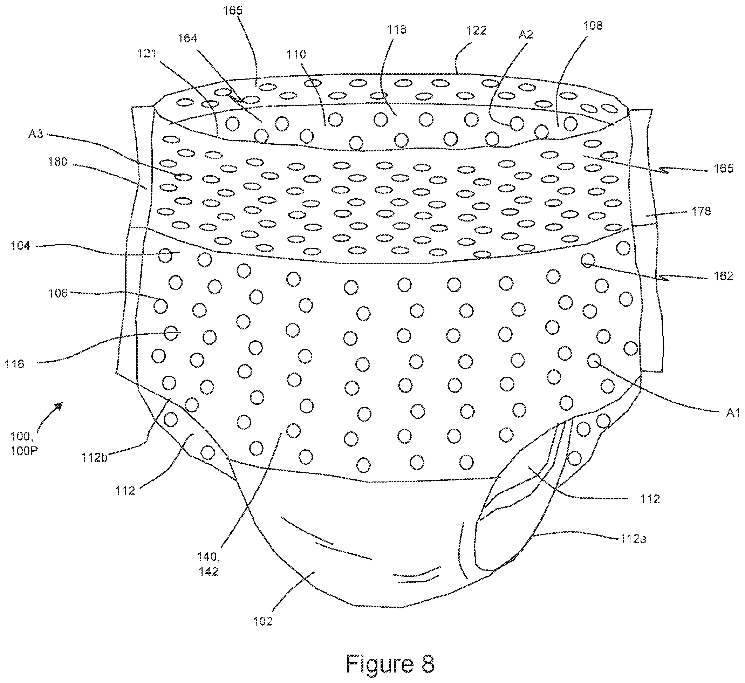

It is also to be appreciated that components of the first elastic belt 106 and/or second elastic belt 108 may comprise additional features, such as apertures arranged in various ways. For example, the first substrate 162, second substrate 164, and/or panel layer 165 may comprise apertures. Such apertures may be arranged in same or different patterns and may comprise the same or different sizes. In some configurations, apertures in the first substrate 162, second substrate 164, and/or panel layer 165 may define an area of about 0.030 mm$^2$ to about 51.000 mm$^2$, specifically reciting all 0.001 mm$^2$ increments within the above-recited range and all ranges formed therein or thereby. In addition, apertures may be positioned within bonded regions and/or within unbonded regions of the first substrate 162, second substrate 164, and/or panel layer 165. Figure 8 shows generic examples of first apertures A1, second apertures A2, and third apertures A3 that may be arranged in first, second, and third patterns and/or first, second, and third sizes, respectively on first substrates, second substrates, and/or panel layers on the first elastic belt 106 and/or the second elastic belt 108. It is to be appreciated that the apertures may be arranged in various ways and may be arranged in various different components of the first elastic belt 106 and/or the second elastic belt 108.

With reference to Figure 8, in some configurations, the first substrate 162 may comprise first apertures, the second substrate 164 may comprise second apertures, and the panel layer 165 may comprise third apertures. In some configurations, the garment facing layer 165c of the panel layer 165 and/or the wearer facing layer 165d of the panel 165 may comprise the third apertures. In some configurations, the first apertures and the second apertures may be at least partially aligned and overlapping to define apertures that extend through both the first substrate 162 and the second substrate 164 in an assembled first elastic belt 106 and/or second elastic belt 108. In some configurations, the first apertures and the third apertures may be at least partially aligned and overlapping to define apertures that extend through both the first substrate 162 and the panel layer 165 in an assembled first elastic belt 106 and/or second elastic belt 108. In some configurations, the second apertures and the third apertures may be at least partially aligned and overlapping to define apertures that extend through both the second substrate 164 and the panel layer 165 in an assembled first elastic belt 106 and/or second elastic belt 108. In some configurations, the first apertures, the second apertures, and the third apertures may be at least partially aligned and overlapping to define apertures that extend through all of the first substrate 162, the second substrate 164, and the panel layer 165 in an assembled first elastic belt 106 and/or second elastic belt 108. In some configurations, the first apertures may define a first size, a first shape, and a first pattern; the second apertures may define a second size, a second shape, and a second pattern; and the third apertures may define a third size, a third shape, and a third pattern. As such, at least one of the first size, the first shape, and the first pattern may be respectively the same or different as at least one of the second size, the second shape, and the second pattern. In some configurations, at least one of the first size, the first shape, and the first pattern may be respectively the same or different as at least one of the third size, the third shape, and the third pattern. And in some configurations, at least one of the second size, the second shape, and the second pattern may be respectively the same or different as at least one of the third size, the third shape, and the third pattern. It is also to be appreciated that at least some of the elastic strands 168 may extend across some of the first apertures, second apertures, and/or third apertures.

It is to be appreciated that the first elastic belt 106 and/or the second elastic belt 108 may include various configurations of belt elastic materials 167 arranged in relation to each other and to the first substrate 162, second substrate 164, and panel layer 165. As discussed above, the elastic material 167 may include configurations of one or more elastic elements such as strands, ribbons, films, or panels positioned in various arrangements. In some configurations, the elastic material 167 may comprise various elastics, elastic features and arrangements, and processes for assembly, such as described in 2018/0168889 A1; 2018/0168874 A1; 2018/0168875 A1; 2018/0168890 A1; 2018/0168887 A1; 2018/0168892 A1; 2018/0168876 A1; 2018/0168891 A1; 2019/0298586 A1; 2019/0070042 A1; 2018/0168878 A1; 2018/0168877 A1; 2018/0168880 A1; 2018/0170027 A1; 2018/0169964 A1; 2018/0168879 A1; 2018/0170026 A1; and 2019/0070041 A1 as well as U.S. Patent Application Nos. 62/989,059 and 62/984,837. It is also to be appreciated the elastic materials 167 herein may be configured with identical or different colors in various different locations on the first elastic belt 106 and/or the second elastic belt 108.

In some configurations, the elastic material 167 may be configured as elastic strands 168 disposed at a constant interval in the longitudinal direction. In other embodiments, the elastic strands 168 may be disposed at different intervals in the longitudinal direction. In some configurations, the elastic material 167 in a stretched condition may be interposed and joined between uncontracted substrate layers. When the elastic material 167 is relaxed, the elastic material 167 returns to an unstretched condition and contracts the substrate layers. The elastic material 167 may provide a desired variation of contraction force in the area of the ring-like elastic belt. It is to be appreciated that the chassis 102 and elastic belts 106, 108 may be configured in different ways other than as depicted in attached Figures. It is also to be appreciated that the elastic material 167 material may be joined to the substrates continuously or intermittently along the interface between the elastic material 167 material and the substrates.

As discussed above for example with reference to Figures 1-6D, the elastic material 167 discussed herein may be in the form of elastic strands 168. It is to be appreciated that the first elastic belt 106 and/or second elastic belt 108 may be configured to include various quantities of elastic strands 168. In some configurations, the first elastic belt 106 and/or second elastic belt 108 may comprise from about 100 to about 1500 elastic strands 168. It is also to be appreciated that elastic strands 168 herein may comprise various Dtex values, strand spacing values, and pre-strain values and such elastic strands 168 may utilized with other elastic strands to create first and second elastic belts 106, 108 comprising elastic strands 168 in various combinations of Dtex values, strand spacing values, and pre-strain values. For example, in some configurations, the Average-Dtex of one or more elastic strands 168 may be greater than 500. In some configurations, the Average-Dtex of one or more elastic strands 168 may be from about 10 to about 500, specifically reciting all 1 Dtex increments within the above-recited range and all ranges formed therein or thereby. In some configurations, a plurality of elastic strands 168 may comprise an Average-Strand-Spacing of less than or equal to 4 mm. In some configurations, a plurality of elastic strands 168 may comprise an Average-Strand-Spacing from about 0.25 mm to about 4 mm, specifically reciting all 0.01 mm increments within the above-recited range and all ranges formed therein or thereby. In some configurations, a plurality of elastic strands 168 may comprise an Average-Strand-Spacing of greater than 4 mm. In some configurations, the Average-Pre-Strain of each of a plurality of elastic strands may be from about 50% to about 400%, specifically reciting all 1% increments within the above-recited range and all ranges formed therein or thereby.

With reference to the first elastic belt 106 and/or the second elastic belt 108 configurations disclosed herein, it is to be appreciated that the elastic strands may arranged in various positions with various Average-Dtex values, Average-Pre-Strain values, and various Average-Strand-Spacing values. For example, with reference to Figures 1-6D, in some configurations, the first elastic belt 106 and/or the second elastic belt 108 may include the first substrate 162 connected with the second substrate 164, and the panel layer 165 connected with either or both the first substrate 162 and the second substrate 164. Elastic material 167 may be positioned between and connected with first substrate 162 and the second substrate 164. In addition, elastic material 167 may be positioned between and connected with the panel layer 165 and the first substrate 162 and/or the second substrate 164. Further, in some configurations, elastic material 167 may be positioned within the garment facing layer 165c and wearer facing layer 165d of the panel layer 165. As previously mentioned, the elastic material 167 may comprise elastic strands 168. As such, the elastic strands 168 may be grouped in pluralities of elastic strands 168. In turn, one or more pluralities of elastic strands 168 may be positioned in various locations between the first substrate 162 and the second substrate 164; in various locations between the panel layer 165 and the first substrate 162; in various locations between the panel layer 165 and the second substrate 164; and/or in various locations between the garment facing layer 165c and wearer facing layer 165d of the panel layer 165. For example, in some configurations, a plurality of elastic strands 168 may be positioned adjacent the distal edge 162a of the first substrate 162 and/or adjacent the distal edge 164a of the second substrate 164. In some configurations, a plurality of elastic strands 168 may be positioned adjacent the proximal edge 162b of the first substrate 162 and/or adjacent the proximal edge 164b of the second substrate 164.

n some configurations, a first plurality of elastic strands may comprise a first Average-Pre-Strain from about 75% to about 300%, and a second plurality of elastic strands may comprise a second Average-Pre-Strain that is greater than first Average-Pre-Strain. In some configurations, a first plurality of elastic strands comprises an Average-Strand-Spacing from about 0.25 mm to about 4 mm and an Average-Dtex from about 10 to about 500; and a second plurality of elastic strands may comprise an Average-Strand-Spacing greater than about 4 mm and an Average-Dtex greater than about 450.

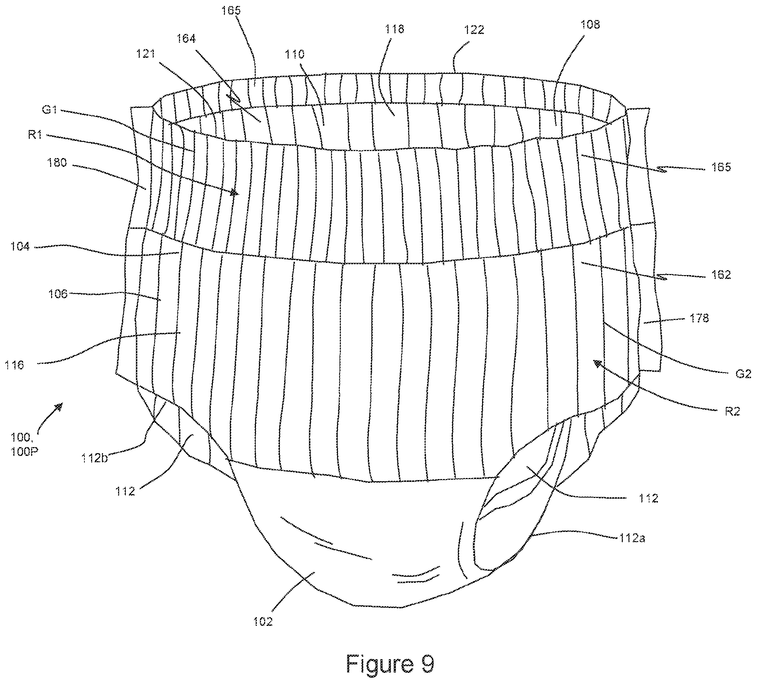

n some configurations of the first elastic belt 106 and/or the second elastic belt 108, when the elastic material 167 is contracted, the first substrate 162, the second substrate 164, and the panel layer 165 may each comprise longitudinally extending gathers. Figure 9 shows generic examples of first gathers G1 and second gathers G2 that may be arranged to comprise various Rugosity Wavelengths and/or Rugosity Frequencies. For example, in some configurations, the first elastic belt 106 and/or the second elastic belt 108 may comprise a first region R1 that includes the panel layer 165. In addition, the first elastic belt 106 and/or the second elastic belt 108 may comprise a second region R2 outside the first region, wherein the second region does not include the panel layer 165. In turn, when the elastic material 167 is contracted, the first region R1 and the second region R2 each comprise longitudinally extending gathers. For example, the first region R1 may comprise first gathers G1 and the second region R2 may comprise second gathers G2. As such, the first region R1 may comprise a first Rugosity Frequency and a first Rugosity Wavelength, and the second region R2 comprises a second Rugosity Frequency and a second Rugosity Wavelength. In some configurations, the first Rugosity Frequency is not equal to the second Rugosity Frequency, and in some configurations, the first Rugosity Wavelength is not equal to the second Rugosity Wavelength. For example, the first Rugosity Frequency may be less than the second Rugosity Frequency.

t is to be appreciated that the first and second Rugosity Frequencies may have various values. For example, in some configurations, at least one of the first Rugosity Frequency and the second Rugosity Frequency may be from about 0.40 to about 0.59, specifically reciting all 0.01 increments within the above-recited range and all ranges formed therein or thereby. In some configurations, at least one of the first Rugosity Frequency and the second Rugosity Frequency may be from about 0.60 to about 2, specifically reciting all 0.01 increments within the above-recited range and all ranges formed therein or thereby. It is also to be appreciated that the first and second Rugosity Wavelengths may have various values. In another example, at least one of the first Rugosity Wavelength and the second Rugosity Wavelength may be from about 0.50mm to about 1.65mm, specifically reciting all 0.01mm increments within the above-recited range and all ranges formed therein or thereby. In some configurations, at least one of the first Rugosity Wavelength and the second Rugosity Wavelength may be from about 1.70mm to about 2.50mm, specifically reciting all 0.01mm increments within the above-recited range and all ranges formed therein or thereby.

t is also to be appreciated that second region R2 may be configured to comprise a plurality of Rugosity Frequencies and Rugosity Wavelengths. For example, the second region R2 may comprises a third Rugosity Frequency and a third Rugosity Wavelength. In some configurations, the third Rugosity Frequency is not equal to the second Rugosity Frequency and/or the first Rugosity Frequency. In some configurations, the third Rugosity Wavelength is not equal to the second Rugosity Wavelength and/or the first Rugosity Wavelength.

As previously mentioned, the first elastic belt 106 and/or the second elastic belt 108 may be constructed to comprise additional desirable features, such as for example, percent contact area and/or height values. In some configurations, at least one of the first elastic belt 106 and the second elastic belt 108 may comprise a Percent Contact Area of at least one of: greater than about 11% at 100 um, greater than about 28% at 200 um, and greater than about 51% at 300 um. And in some configurations, at least one of the first elastic belt 106 and the second elastic belt 108 may comprise a 2%-98% Height Value of <1.6 mm

AVERAGE DECITEX (AVERAGE-DTEX)

The Average Decitex Method is used to calculate the Average-Dtex on a length-weighted basis for elastic fibers present in an entire article, or in a specimen of interest extracted from an article. The decitex value is the mass in grams of a fiber present in 10,000 meters of that material in the relaxed state. The decitex value of elastic fibers or elastic laminates containing elastic fibers is often reported by manufacturers as part of a specification for an elastic fiber or an elastic laminate including elastic fibers. The Average-Dtex is to be calculated from these specifications if available. Alternatively, if these specified values are not known, the decitex value of an individual elastic fiber is measured by determining the cross-sectional area of a fiber in a relaxed state via a suitable microscopy technique such as scanning electron microscopy (SEM), determining the composition of the fiber via Fourier Transform Infrared (FT-IR) spectroscopy, and then using a literature value for density of the composition to calculate the mass in grams of the fiber present in 10,000 meters of the fiber. The manufacturer-provided or experimentally measured decitex values for the individual elastic fibers removed from an entire article, or specimen extracted from an article, are used in the expression below in which the length-weighted average of decitex value among elastic fibers present is determined.

he lengths of elastic fibers present in an article or specimen extracted from an article is calculated from overall dimensions of and the elastic fiber pre-strain ratio associated with components of the article with these or the specimen, respectively, if known. Alternatively, dimensions and/or elastic fiber pre-strain ratios are not known, an absorbent article or specimen extracted from an absorbent article is disassembled and all elastic fibers are removed. This disassembly can be done, for example, with gentle heating to soften adhesives, with a cryogenic spray (e.g. Quick-Freeze, Miller-Stephenson Company, Danbury, CT), or with an appropriate solvent that will remove adhesive but not swell, alter, or destroy elastic fibers. The length of each elastic fiber in its relaxed state is measured and recorded in millimeters (mm) to the nearest mm.

Calculation of Average-Dtex

For each of the individual elastic fibers $f_i$ of relaxed length $L_i$ and fiber decitex value $d_i$ (obtained either from the manufacturer's specifications or measured experimentally) present in an absorbent article, or specimen extracted from an absorbent article, the Average-Dtex for that absorbent article or specimen extracted from an absorbent article is defined as:

$$\text{Average-Dtex} = \frac{\sum_{i=1}^{n}(L_i \times d_i)}{\sum_{i=1}^{n} L_i}$$

where n is the total number of elastic fibers present in an absorbent article or specimen extracted from an absorbent article. The Average-Dtex is reported to the nearest integer value of decitex (grams per 10 000 m).

If the decitex value of any individual fiber is not known from specifications, it is experimentally determined as described below, and the resulting fiber decitex value(s) are used in the above equation to determine Average-Dtex.

Experimental Determination of Decitex Value for a Fiber

For each of the elastic fibers removed from an absorbent article or specimen extracted from an absorbent article according to the procedure described above, the length of each elastic fiber $L_k$ in its relaxed state is measured and recorded in millimeters (mm) to the nearest mm. Each elastic fiber is analyzed via FT-IR spectroscopy to determine its composition, and its density $\rho_k$ is determined from available literature values. Finally, each fiber is analyzed via SEM. The fiber is cut in three approximately equal locations perpendicularly along its length with a sharp blade to create a clean cross-section for SEM analysis. Three fiber segments with these cross sections exposed are mounted on an SEM sample holder in a relaxed state, sputter coated with gold, introduced into an SEM for analysis, and imaged at a resolution sufficient to clearly elucidate fiber cross sections. Fiber cross sections are oriented as perpendicular as possible to the detector to minimize any oblique distortion in the measured cross sections. Fiber cross sections may vary in shape, and some fibers may consist of a plurality of individual filaments. Regardless, the area of each of the three fiber cross sections is determined (for example, using diameters for round fibers, major and minor axes for elliptical fibers, and image analysis for more complicated shapes), and the average of the three areas $a_k$ for the elastic fiber, in units of micrometers squared ($\mu m^2$), is recorded to the nearest 0.1 $\mu m^2$. The decitex $d_k$ of the kth elastic fiber measured is calculated by:

$$ d_k = 10\ 000\ \text{m}\ \times\ a_k \times \rho_k \times 10^{-6} $$

where $d_k$ is in units of grams (per calculated 10,000 meter length), $a_k$ is in units of $\mu m^2$, and $\rho_k$ is in units of grams per cubic centimeter (g/cm$^3$). For any elastic fiber analyzed, the experimentally determined $L_k$ and $d_k$ values are subsequently used in the expression above for Average-Dtex.

AVERAGE-STRAND-SPACING

Using a ruler calibrated against a certified NIST ruler and accurate to 0.5 mm, measure the distance between the two distal strands within a section to the nearest 0.5 mm, and then divide by the number of strands in that section - 1

$$ \text{Average-Strand-Spacing} = \text{d/(n-1)} $$

where n>1
report to the nearest 0.1 mm.

AVERAGE-PRE-STRAIN

The Average-Pre-Strain of a specimen are measured on a constant rate of extension tensile tester (a suitable instrument is the MTS Insight using Testworks 4.0 Software, as available from MTS Systems Corp., Eden Prairie, MN) using a load cell for which the forces measured are within 1% to 90% of the limit of the cell. Articles are conditioned at 23 °C $\pm$ 2 C° and 50% $\pm$ 2% relative humidity for 2 hours prior to analysis and then tested under the same environmental conditions.
Program the tensile tester to perform an elongation to break after an initial gage length adjustment. First raise the cross head at 10 mm/min up to a force of 0.05N. Set the current gage to the adjusted gage length. Raise the crosshead at a rate of 100 mm/min until the specimen breaks (force drops 20% after maximum peak force). Return the cross head to its original position. Force and extension data is acquired at a rate of 100 Hz throughout the experiment.
Set the nominal gage length to 40 mm using a calibrated caliper block and zero the crosshead. Insert the specimen into the upper grip such that the middle of the test strip is positioned 20 mm below the grip. The specimen may be folded perpendicular to the pull axis, and placed in the grip to achieve this position. After the grip is closed the excess material can be trimmed. Insert the specimen into the lower grips and close. Once again, the strip can be folded, and then trimmed after the grip is closed. Zero the load cell. The specimen should have a minimal slack but less than 0.05 N of force on the load cell. Start the test program.
From the data construct a Force (N) verses Extension (mm). The Average-Pre-Strain is calculated from the bend in the curve corresponding to the extension at which the nonwovens in the elastic are engaged. Plot two lines, corresponding to the region of the curve before the bend (primarily the elastics), and the region after the bend (primarily the nonwovens). Read the extension at which these two lines intersect, and calculate the % Pre-Strain from the extension and the corrected gage length. Record as % Pre-strain 0.1%. Calculate the arithmetic mean of three replicate samples for each elastomeric laminate and Average-Pre-Strain to the nearest 0.1%.

SURFACE TOPOGRAPHY (PERCENT CONTACT AREA, RUGOSITY FREQUENCY, RUGOSITY WAVELENGTH AND 2-98% HEIGHT VALUE)

In the Surface Topography Method, an elastic laminate specimen is removed from an absorbent article and extended across and in contact with the convex surface of a transparent horizontal cylindrical tubing segment, allowing the areal surface topology of the body facing side of the laminate to be measured through the transparent tubing segment using optical profilometry. The 3D surface data are then sampled and processed to extract several parameters that describe the percent contact area and height of the elastic laminate specimen surface as well as the frequency and wavelength of its associated rugosities. All sample preparation and testing is performed in a conditioned room maintained at about 23 ± 2 °C and about 50 ± 2 % relative humidity, and samples are equilibrated in this environment for at least 24 hours prior to testing.

Sample Preparation

Each elastic laminate specimen extracted from an article is mounted on a horizontal tubing segment as described below. The tubing segment is cut from a sufficient length of optically clear, colorless cast acrylic cylindrical tubing having an outer diameter of 8.0 inches (203 mm) and a wall thickness of 0.1875 inches (4.76 mm). The segment has a dimension of 4.0 inches (102 mm) along an axis parallel to the central cylindrical axis of the parent tubing and a circumferential outer arc length of 5.5 inches (140 mm).

The elastic laminate specimen is extended in its primary stretch direction to a width of 120% of the original, for example a sample having an original width of 100 mm would be extended to a width of 120 mm for testing. In this extended state, the extended elastic laminate specimen is oriented such that its body-facing surface is in contact with the convex surface of the tubing segment and that the axis of extension is oriented around the circumference of the tubing segment. The extended laminate is secured at both ends to the transparent tubing segment such that the body-facing surface of the laminate is viewable through the concave side of the transparent tubing segment.

Five replicate elastic laminate specimens are isolated and prepared in this way from five equivalent absorbent articles for analysis.

3D surface image acquisition

A three-dimensional (3D) surface topography image of the body facing surface of the extended elastic laminate specimen is obtained using a DLP-based, structured-light 3D surface topography measurement system (a suitable surface topography measurement system is the MikroCAD Premium instrument commercially available from LMI Technologies Inc., Vancouver, Canada, or equivalent). The system includes the following main components: a) a Digital Light Processing (DLP) projector with direct digital controlled micro-mirrors; b) a CCD camera with at least a 1600 x 1200 pixel resolution; c) projection optics adapted to a measuring area of at least 60 mm x 45 mm; d) recording optics adapted to a measuring area of 60 mm x 45 mm; e) a table tripod based on a small hard stone plate; f) a blue LED light source; g) a measuring, control, and evaluation computer running surface texture analysis software (a suitable software is MikroCAD software with Mountains Map technology, or equivalent); and h) calibration plates for lateral (XY) and vertical (Z) calibration available from the vendor.

The optical 3D surface topography measurement system measures the surface height of a sample using the digital micro-mirror pattern fringe projection technique. The nature of this pattern projection technique allows the surface topography of a specimen to be interrogated through a transparent material. The result of the measurement is a 3D data set of surface height (defined as the Z-axis) versus displacement in the horizontal (XY) plane. This 3D data set can also be thought of as an image in which every pixel in the image there is associated an XY displacement, and the value of the pixel is the recorded Z-axis height value. The system has a field of view of 60 x 45 mm with an XY pixel resolution of approximately 37 microns, and a height resolution of 0.5 microns, with a total possible height range of 32 mm.

The instrument is calibrated according to manufacturer's specifications using the calibration plates for lateral (XY plane) and vertical (Z-axis) available from the vendor.

The elastic laminate specimen mounted on the transparent tubing segment is positioned with the concave surface of the tubing segment surface facing upward so that the body facing surface is facing upward and visible through the transparent material. The tubing segment is placed on a stand such that the convex (downward-facing) specimen surface in the region to be analyzed is suspended freely and not resting on a surface. The tubing segment is oriented such that its circumferential direction (that direction or axis along which the laminate is stretched) is centered and perpendicular relative to the long axis of the camera's field of view (or either of the central axes if the field of view is square). A 3D surface topology image of the elastic laminate specimen is collected by following the instrument manufacturer's recommended measurement procedures, which may include focusing the measurement system and performing a brightness adjustment. No pre-filtering options are used. The collected height image file is saved to the evaluation computer running the surface texture analysis software.

If the field of view of the 3D surface topography measurement system exceeds the evaluation region on the elastic laminate specimen the image may be cropped to remove extraneous areas and retain a rectangular field of view of the relevant portion, while maintaining the XY resolution, prior to performing the analysis.

3D surface image analysis

The 3D surface topography image is opened in the surface texture analysis software. The following filtering procedure is then performed on each image: 1) removal of invalid or non-measured points; 2) a 5x5 pixel median filter to remove noise; 3) a 5x5 pixel mean filter to smooth the surface; and 4) subtraction of a two-dimensional, second-order polynomial (determined via least-squares fit of the surface topology image) to remove the general form and flatten the surface. The second-order polynomial is defined by the following equation:

$$f(x, y) = c_1 + c_2 x + c_3 y + c_4 x^2 + c_5 y^2 + c_6 xy$$

Each data set that has been processed to this point as described above is referred to as a "preprocessed specimen data set." The highest points of the resulting topology image correspond to those areas in contact with the convex surface of the tubing segment, and the lowest points are those points most distal below the convex surface of the tubing segment.

CONTACT SURFACE AREAS AND 2-98% HEIGHT VALUE

For each of the 3D surface topography images of the five replicate specimens, the following analysis is performed on preprocessed specimen data sets. The Percent Surface Contact Area and 2-98% Height measurements are derived from the Areal Material Ratio (Abbott-Firestone) curve described in the ISO 13565-2:1996 standard extrapolated to surfaces. This curve is the cumulative curve of the surface height distribution histogram versus the range of surface heights measured. A material ratio is the ratio, expressed as a percent, of the area corresponding to points with heights equal to or above an intersecting plane passing through the surface at a given height, or cut depth, to the cross-sectional area of the evaluation region (field of view area). The height at a material ratio of 2% is initially identified. A cut depth of 100 $\mu$m below this height is then identified, and the material ratio at this depth is recorded as the Percent Surface Contact Area at 100 $\mu$m. This procedure is repeated at a cut depth of 200 $\mu$m and 300 $\mu$m below the identified height at a material ratio of 2%, and the material ratio at these depths are recorded as the Percent Surface Contact Area at 200 $\mu$m and the Percent Surface Contact Area at 300 $\mu$m respectively. All of the Percent Contact Area values are recorded to the nearest 0.1%.
The 2-98% Height of the specimen surface is defined as the difference in heights between two material ratios that exclude a small percentage of the highest peaks and lowest valleys. The 2-98% Height of the specimen surface is the height between the two cutting depths corresponding to a material ratio value of 2% to the material ratio of 98%, and is recorded to the nearest 0.01 mm.

Rugosity Frequency and Rugosity Wavelength

The preprocessed 3D surface topology images for each specimen are subjected to Fourier transform spatial frequency analysis to determine Rugosity Frequency and Rugosity Wavelength.
Each 3D surface topology image is deconstructed into individual line profiles by isolating each entire row of single data points that run in the dimension parallel to the elastic strands (if present and evident) of the elastic laminate, or, more generally, perpendicular to the rugosity exhibited by the elastic laminate in the relaxed state. These line profiles are therefore data sets in the form of height (in millimeters) versus distance (in millimeters).
For each replicate 3D surface topology image deconstructed, each line profile is mean centered, and a fast Fourier transform (FFT) is applied to calculate the frequency amplitude spectrum of each line profile. The Fourier transform amplitude versus spatial frequency spectra of all extracted line profiles are averaged, and the resulting average amplitude versus spatial frequency spectrum is defined as F(1/d), where 1/d is reciprocal distance in units of mm$^{-1}$. Finally, the function P(1/d) = d $\times$ F$^2$(1/d), the spatial frequency power spectral density with a prefactor of distance d to correct for the expected 1/d noise, is plotted versus 1/d. The value of reciprocal distance 1/d at which P(1/d) is at a maximum is defined as the Rugosity Frequency and is recorded in units of mm$^{-1}$ to the nearest 0.001 mm$^{-1}$. The reciprocal of the Rugosity Frequency is defined as the Rugosity Wavelength and is recorded in units of mm to the nearest 0.01 mm.

Reporting of Method Parameters

After the 3D surface image analysis described above is performed on 3D surface topology images of all five specimen replicates, the following output parameters are defined and reported. The arithmetic mean of all five Percent Surface Contact Area at 100 $\mu$m measurements is defined as the Average Percent Surface Contact Area at 100 $\mu$m and is reported to the nearest 0.1%. The arithmetic mean of all five Percent Surface Contact Area at 200 $\mu$m measurements is defined as the Average Percent Surface Contact Area at 200 $\mu$m and is reported to the nearest 0.1%. The arithmetic mean of all five Percent Surface Contact Area at 300 $\mu$m measurements is defined as the Average Percent Surface Contact Area

at 300 $\mu$m and is reported to the nearest 0.1%. The arithmetic mean of all five 2-98% Height measurements is defined as the Average 2-98% Height and is reported in units of mm to the nearest 0.01 mm. The arithmetic mean of all five Rugosity Frequency measurements is defined as the Average Rugosity Frequency and is reported in units of mm to the nearest 0.001 mm$^{-1}$. The arithmetic mean of all five Rugosity Wavelength measurements is defined as the Average Rugosity Wavelength and is reported in units of mm to the nearest 0.01 mm.

**Claims**

1. An absorbent article comprising:

   a chassis (102) comprising a topsheet (138), a backsheet (136), and an absorbent core (140) positioned between the topsheet (138) and the backsheet (136), the chassis (102) further comprising a first end region (116a) and a second end region (118a) longitudinally separated from the first end region (116a) by a crotch region (119);
   a first belt (106) connected with the first end region (116a) of the chassis (102);
   a second belt (108) connected with the second end region (118a) of the chassis (102), wherein laterally opposing end portions (108a, 108b) of the second belt (108) are connected with laterally opposing end portions (106a, 106b) of the first belt (106) to form a waist opening (110);
   wherein at least one of the first belt (106) and the second belt (108) comprises:

   a distal edge (121, 122) extending along a portion of the waist opening (110);
   an elastic material (167) positioned between and connected with a first substrate (162) and a second substrate (164), the elastic material (167) comprising a first plurality of elastic strands (168) positioned between and connected with the first substrate (162) and the second substrate (164), wherein the first plurality of elastic strands (168) comprises an Average- Strand- Spacing from about 0.25 mm to about 4 mm and an Average-Dtex from about 10 to about 500;
   wherein the first substrate (162) and the second substrate (164) each comprise a wearer facing surface (162d, 164d), a garment facing surface (162c, 164c); and
   a panel layer (165) connected with the wearer surface (164d) of the second substrate (164), the panel layer (165) comprising a first lateral edge (165a) and a second lateral edge (165b), wherein the first lateral edge (165a) is positioned longitudinally outboard of the second lateral edge (165b)
   wherein the panel layer (165) is a laminate; and
   wherein the laminate comprises a second elastic material.

2. The absorbent article of claim 1, wherein the first lateral edge (165a) of the panel layer (165) is positioned coterminous with the distal edge (121, 122) of at least one of the first belt (106) and the second belt (108).

3. The absorbent article of claim 1, wherein the first lateral edge (165a) of the panel layer (165) is positioned longitudinally inboard of the distal edge (121, 122) of at least one of the first belt (106) and the second belt (108).

4. The absorbent article of claim 1, wherein at least one of the first substrate (162) and the second substrate (164) comprises a proximal edge (162b, 164b) extending across the backsheet (136).

5. The absorbent article according to any one of the preceding claims, wherein the elastic material (167) comprises elastic film.

6. The absorbent article according to any one of the preceding claims, wherein the elastic material (167) further comprises a second plurality of elastic strands (168), wherein the second plurality of elastic strands comprises an Average- Strand- Spacing greater than about 4 mm and an Average-Dtex greater than about 450.

7. The absorbent article of claim 6, wherein at least one of the first plurality of elastic strands (168) and the second plurality of elastic strands (168) are bonded with the panel layer (165).

8. The absorbent article according to any one of the preceding claims, further comprising:

   a first region (RI) of at least one of the first belt (106) and the second belt (108) that comprises the panel layer (165);
   a second region (R2) of at least one of the first belt (106) and the second belt (108) outside the first region (RI) that does not comprise the panel layer (165); and

wherein when the elastic material (167) is contracted, the first region (RI) and the second region (R2) each comprise longitudinally extending gathers, wherein the first region (RI) comprises a first Rugosity Frequency measured according to the method set out herein and a first Rugosity Wavelength measured according to the method set out herein, and the second region (R2) comprises a second Rugosity Frequency measured according to the method set out herein and a second Rugosity Wavelength measured according to the method set out herein.

9. The absorbent article of claim 8, wherein the first Rugosity Frequency is not equal to the second Rugosity Frequency.

10. The absorbent article of claim 9, wherein at least one of the first Rugosity Frequency and the second Rugosity Frequency is from about 0.40 to about 0.59, and wherein at least one of the first Rugosity Frequency and the second Rugosity Frequency is from about 0.60 to about 2.

11. The absorbent article of claim 10, wherein at least one of the first Rugosity Wavelength and the second Rugosity Wavelength is from about 0.50mm to about 1,65mm, and wherein at least one of the first Rugosity Wavelength and the second Rugosity Wavelength is from about 1.70mm to about 2.50mm.

12. The absorbent article according to any one of the preceding claims, wherein the first substrate (162) defines a first lateral width W1 between a first longitudinal edge and a second longitudinal edge; the second substrate (164) defines a second lateral width W2 between a first longitudinal edge and a second longitudinal edge; and the panel layer defines a third lateral width W3 between a first longitudinal edge and a second longitudinal edge, wherein the third lateral width W3 is equal to at least one of the first lateral width W 1 and the second lateral width W2, and preferably wherein the third lateral width W3 is less than at least one of the first lateral width W1 and the second lateral width W2.

13. The absorbent article according to any one of the preceding claims, wherein panel layer (165) covers a portion of the first end region (116a) of the chassis (102).

## Patentansprüche

1. Absorptionsartikel, umfassend:

   eine Grundeinheit (102), umfassend eine Oberschicht (138), eine Unterschicht (136) und einen Absorptionskern (140), der zwischen der Oberschicht (138) und der Unterschicht (136) angeordnet ist, die Grundeinheit (102) ferner umfassend einen ersten Endbereich (116a) und einen zweiten Endbereich (118a), der in Längsrichtung von dem ersten Endbereich (116a) durch einen Schrittbereich (119) getrennt ist;
   einen ersten Bund (106), der mit dem ersten Endbereich (116a) der Grundeinheit (102) verbunden ist;
   einen zweiten Bund (108), der mit dem zweiten Endbereich (118a) der Grundeinheit (102) verbunden ist, wobei einander seitlich entgegengesetzte Endabschnitte (108a, 108b) des zweiten Bunds (108) mit einander seitlich entgegengesetzten Endabschnitten (106a, 106b) des ersten Bunds (106) verbunden sind, um eine Taillenöffnung (110) auszubilden;
   wobei mindestens einer des ersten Bunds (106) und des zweiten Bunds (108) umfasst:

   einen distalen Rand (121, 122), der sich entlang eines Abschnitts der Taillenöffnung (110) erstreckt;
   ein elastisches Material (167), das zwischen einem ersten Substrat (162) und einem zweiten Substrat (164) positioniert und mit diesen verbunden ist, das elastische Material (167) umfassend eine erste Vielzahl von elastischen Strängen (168), die zwischen dem ersten Substrat (162) und dem zweiten Substrat (164) positioniert und mit diesen verbunden sind, wobei die erste Vielzahl von elastischen Strängen (168) einen durchschnittlichen Strangabstand von etwa 0,25 mm bis etwa 4 mm und einen durchschnittlichen Dtex von etwa 10 bis etwa 500 umfasst;
   wobei das erste Substrat (162) und das zweite Substrat (164) jeweils eine trägerseitige Oberfläche (162d, 164d) und eine bekleidungsseitige Oberfläche (162c, 164c) umfassen; und
   eine Feldschicht (165), die mit der Trägeroberfläche (164d) des zweiten Substrats (164) verbunden ist, die Feldschicht (165) umfassend einen ersten Seitenrand (165a) und einen zweiten Seitenrand (165b), wobei der erste Seitenrand (165a) in Längsrichtung außerhalb des zweiten Seitenrands (165b) positioniert ist.
   wobei die Feldschicht (165) ein Laminat ist; und
   wobei das Laminat ein zweites elastisches Material umfasst.

2. Absorptionsartikel nach Anspruch 1, wobei der erste Seitenrand (165a) der Feldschicht (165) deckungsgleich mit dem distalen Rand (121, 122) von mindestens einem des ersten Bunds (106) und des zweiten Bunds (108) positioniert ist.

3. Absorptionsartikel nach Anspruch 1, wobei der erste Seitenrand (165a) der Feldschicht (165) in Längsrichtung innerhalb des distalen Rands (121, 122) von mindestens einem des ersten Bunds (106) und des zweiten Bunds (108) positioniert ist.

4. Absorptionsartikel nach Anspruch 1, wobei mindestens eines des ersten Substrats (162) und des zweiten Substrats (164) einen proximalen Rand (162b, 164b) umfasst, die sich über die Unterschicht (136) hinweg erstreckt.

5. Absorptionsartikel nach einem der vorstehenden Ansprüche, wobei das elastische Material (167) elastische Folie umfasst.

6. Absorptionsartikel nach einem der vorstehenden Ansprüche, wobei das elastische Material (167) ferner eine zweite Vielzahl von elastischen Strängen (168) umfasst, wobei die zweite Vielzahl von elastischen Strängen einen durchschnittlichen Strangabstand von mehr als etwa 4 mm und einen durchschnittlichen Dtex von mehr als etwa 450 umfasst.

7. Absorptionsartikel nach Anspruch 6, wobei mindestens einer der ersten Vielzahl von elastischen Strängen (168) und der zweiten Vielzahl von elastischen Strängen (168) an die Feldschicht (165) gebunden ist.

8. Absorptionsartikel nach einem der vorstehenden Ansprüche, ferner umfassend:

einen ersten Bereich (R1) von mindestens einem des ersten Bands (106) und des zweiten Bands (108), der die Feldschicht (165) umfasst;
einen zweiten Bereich (R2) von mindestens einem des ersten Bands (106) und des zweiten Bands (108) außerhalb des ersten Bereichs (R1), der nicht die Feldschicht (165) umfasst; und
wobei, wenn das elastische Material (167) kontrahiert wird, der erste Bereich (R1) und der zweite Bereich (R2) jeweils sich in Längsrichtung erstreckende Raffungen umfassen, wobei der erste Bereich (R1) eine erste Rauhigkeitsfrequenz, gemessen gemäß dem hierin beschriebenen Verfahren, und eine erste Rauhigkeitswellenlänge, gemessen gemäß dem hierin beschriebenen Verfahren, umfasst, und der zweite Bereich (R2) eine zweite Rauhigkeitsfrequenz, gemessen gemäß dem hierin beschriebenen Verfahren, und eine zweite Rauhigkeitswellenlänge, gemessen gemäß dem hierin beschriebenen Verfahren, umfasst.

9. Absorptionsartikel nach Anspruch 8, wobei die erste Rauhigkeitsfrequenz nicht gleich der zweiten Rauhigkeitsfrequenz ist.

10. Absorptionsartikel nach Anspruch 9, wobei mindestens eine der ersten Rauhigkeitsfrequenz und der zweiten Rauhigkeitsfrequenz von etwa 0,40 bis etwa 0,59 beträgt und wobei mindestens eine der ersten Rauhigkeitsfrequenz und der zweiten Rauhigkeitsfrequenz von etwa 0,60 bis etwa 2 beträgt.

11. Absorptionsartikel nach Anspruch 10, wobei mindestens eine der ersten Rauheitswellenlänge und der zweiten Rauheitswellenlänge von etwa 0,50 mm bis etwa 1,65 mm beträgt und wobei mindestens eine der ersten Rauheitswellenlänge und der zweiten Rauheitswellenlänge von etwa 1,70 mm bis etwa 2,50 mm beträgt.

12. Absorptionsartikel nach einem der vorstehenden Ansprüche, wobei das erste Substrat (162) eine erste seitliche Breite W1 zwischen einem ersten Längsrand und einem zweiten Längsrand definiert; das zweite Substrat (164) eine zweite seitliche Breite W2 zwischen einem ersten Längsrand und einem zweiten Längsrand definiert; und die Feldschicht eine dritte seitliche Breite W3 zwischen einem ersten Längsrand und einem zweiten Längsrand definiert, wobei die dritte seitliche Breite W3 gleich mindestens einer der ersten seitlichen Breite W1 und der zweiten seitlichen Breite W2 ist und vorzugsweise wobei die dritte seitliche Breite W3 geringer als mindestens eine der ersten seitliche Breite W1 und der zweiten seitliche Breite W2 ist.

13. Absorptionsartikel nach einem der vorstehenden Ansprüche, wobei die Feldschicht (165) einen Abschnitt des ersten Endbereichs (116a) der Grundeinheit (102) bedeckt.

**Revendications**

1. Article absorbant comprenant :

un châssis (102) comprenant une feuille de dessus (138), une feuille de fond (136) et une âme absorbante (140) placée entre la feuille de dessus (138) et la feuille de fond (136), le châssis (102) comprenant en outre une première région d'extrémité (116a) et une seconde région d'extrémité (118a) séparée longitudinalement de la première région d'extrémité (116a) par une région d'entrejambe (119) ;
une première ceinture (106) reliée à la première zone d'extrémité (116a) du châssis (102) ;
une seconde ceinture (108) reliée à la seconde région d'extrémité (118a) du châssis (102), dans lequel les parties d'extrémité latéralement opposées (108a, 108b) de la seconde ceinture (108) sont reliées aux parties d'extrémité latéralement opposées (106a, 106b) de la première ceinture (106) pour former une ouverture de taille (110) ;
dans lequel au moins l'une parmi la première ceinture (106) et la seconde ceinture (108) comprend :

un bord distal (121, 122) s'étendant le long d'une partie de l'ouverture de taille (110) ;
un matériau élastique (167) placé entre un premier substrat (162) et un second substrat (164) et relié à ceux-ci, le matériau élastique (167) comprenant une première pluralité de brins élastiques (168) placés entre le premier substrat (162) et le second substrat (164) et reliés à ceux-ci, dans lequel la première pluralité de brins élastiques (168) comprend un espacement moyen de brin compris entre environ 0,25 mm et environ 4 mm et un Dtex moyen compris entre environ 10 et environ 500 ;
dans lequel le premier substrat (162) et le second substrat (164) comprennent chacun une surface orientée vers le porteur (162d, 164d), une surface orientée vers le vêtement (162c, 164c) ; et
une couche de panneau (165) reliée à la surface de porteur (164d) du second substrat (164), la couche de panneau (165) comprenant un premier bord latéral (165a) et un second bord latéral (165b), dans lequel le premier bord latéral (165a) est placé longitudinalement à l'extérieur du second bord latéral (165b).
dans lequel la couche de panneau (165) est un stratifié ; et
dans lequel le stratifié comprend un second matériau élastique.

2. Article absorbant selon la revendication 1, dans lequel le premier bord latéral (165a) de la couche de panneau (165) est placé adjacent au bord distal (121, 122) d'au moins l'une parmi la première ceinture (106) et la seconde ceinture (108).

3. Article absorbant selon la revendication 1, dans lequel le premier bord latéral (165a) de la couche de panneau (165) est placé longitudinalement à l'intérieur du bord distal (121, 122) d'au moins l'une parmi la première ceinture (106) et la seconde ceinture (108).

4. Article absorbant selon la revendication 1, dans lequel au moins l'un parmi le premier substrat (162) et le second substrat (164) comprend un bord proximal (162b, 164b) s'étendant à travers la feuille de fond (136).

5. Article absorbant selon l'une quelconque des revendications précédentes, dans lequel le matériau élastique (167) comprend un film élastique.

6. Article absorbant selon l'une quelconque des revendications précédentes, dans lequel le matériau élastique (167) comprend en outre une seconde pluralité de brins élastiques (168), dans lequel la seconde pluralité de brins élastiques comprend un espacement moyen de brin supérieur à environ 4 mm et un Dtex moyen supérieur à environ 450.

7. Article absorbant selon la revendication 6, dans lequel au moins l'une parmi la première pluralité de brins élastiques (168) et la seconde pluralité de brins élastiques (168) est liée à la couche de panneau (165).

8. Article absorbant selon l'une quelconque des revendications précédentes comprenant en outre :

une première région (R1) d'au moins l'une parmi la première ceinture (106) et la seconde ceinture (108) qui comprend la couche de panneau (165) ;
une seconde région (R2) d'au moins l'une parmi la première ceinture (106) et la seconde ceinture (108) à l'extérieur de la première région (R1) qui ne comprend pas la couche de panneau (165) ; et
dans lequel, lorsque le matériau élastique (167) est contracté, la première région (R1) et la seconde région (R2) comprennent chacune des fronces s'étendant longitudinalement, dans lequel la première région (R1) comprend une première fréquence de rugosité mesurée selon le procédé exposé ici et une première longueur d'onde de rugosité mesurée selon le procédé exposé ici, et la seconde région (R2) comprend une seconde fréquence de rugosité mesurée selon le procédé exposé ici et une seconde longueur d'onde de rugosité mesurée selon le procédé exposé ici.

9. Article absorbant selon la revendication 8, dans lequel la première fréquence de rugosité n'est pas égale à la seconde fréquence de rugosité.

10. Article absorbant selon la revendication 9, dans lequel au moins l'une parmi la première fréquence de rugosité et la seconde fréquence de rugosité est comprise entre environ 0,40 et environ 0,59, et dans lequel au moins l'une parmi la première fréquence de rugosité et la seconde fréquence de rugosité est comprise entre environ 0,60 et environ 2.

11. Article absorbant selon la revendication 10, dans lequel au moins l'une parmi la première longueur d'onde de rugosité et la seconde longueur d'onde de rugosité est comprise entre environ 0,50 mm et environ 1,65 mm, et dans lequel au moins l'une parmi la première longueur d'onde de rugosité et la seconde longueur d'onde de rugosité est comprise entre environ 1,70 mm et environ 2,50 mm.

12. Article absorbant selon l'une quelconque des revendications précédentes, dans lequel le premier substrat (162) définit une première largeur latérale W1 entre un premier bord longitudinal et un second bord longitudinal ; le second substrat (164) définit une deuxième largeur latérale W2 entre un premier bord longitudinal et un second bord longitudinal ; et la couche de panneau définit une troisième largeur latérale W3 entre un premier bord longitudinal et un second bord longitudinal, dans lequel la troisième largeur latérale W3 est égale à au moins l'une parmi la première largeur latérale W1 et la deuxième largeur latérale W2, et de préférence dans lequel la troisième largeur latérale W3 est inférieure à au moins l'une parmi la première largeur latérale W1 et la deuxième largeur latérale W2.

13. Article absorbant selon l'une quelconque des revendications précédentes, dans lequel la couche de panneau (165) recouvre une partie de la première région d'extrémité (116a) du châssis (102).

Figure 1

Figure 2A

Figure 2B

EP 4 243 750 B1

27

Figure 2C

28

Figure 2D

Figure 2E

Figure 3

EP 4 243 750 B1

165

168

167,
168,
169

164,
164d

164c

106
(108)

167,
168

162,
162c

162d

140,
142

132,
138

102

134,
136

Figure 3A

165a

165

167,
168,
169

168

165b

164,
164d

164c

106
(108)

167,
168

162,
162c

162d

140,
142

132,
138

102

134,
136

Figure 3B

Figure 3C

Figure 4B

Figure 4A

Figure 4C

Figure 4D

EP 4 243 750 B1

Figure 4E

Figure 4F

Figure 5A

Figure 5B

Figure 5C

Figure 5D

Figure 6A

Figure 6B

Figure 6D

Figure 6C

Figure 7

Figure 8

Figure 9

**REFERENCES CITED IN THE DESCRIPTION**

**Patent documents cited in the description**

- US 2018168874 A
- US 6120487 A
- US 5167897 A
- US 5360420 A
- US 5599335 A
- US 5643588 A
- US 5674216 A
- US 5702551 A
- US 5968025 A
- US 6107537 A
- US 6118041 A
- US 6153209 A
- US 6410129 B
- US 6426444 B
- US 6586652 B
- US 6627787 B
- US 6617016 B
- US 6825393 B
- US 6861571 B
- US 20130072887 A1
- US 20130211356 A1
- US 20130306226 A1
- US 4940464 A
- US 5092861 A
- US 5246433 A
- US 5569234 A
- US 5897545 A
- US 5957908 A
- US 6120489 A
- US 7569039 B
- US 20030233082 A1
- US 20050107764 A1
- US 20120061016 A1
- US 20120061015 A1
- US 20130255861 A1
- US 20130255862 A1
- US 20130255863 A1
- US 20130255864 A1
- US 20130255865 A1
- US 5628097 A
- US 5916661 A
- US 6545197 B
- US 6107539 B
- US 4610678 A
- US 4673402 A
- US 4888231 A
- US 4834735 A
- US 5562646 A
- US 5669894 A
- US 6790798 A
- US 20040158212 A1
- US 20040097895 A1
- US 3860003 A
- US 4909803 A
- US 4695278 A
- US 4795454 A
- US 4704115 A
- US 20090312730 A1
- US 5681302 A
- US 8186296 B
- US 9265672 B
- US 9248054 B
- US 9295590 B
- US 20140148773 A1
- US 3113225 A
- US 3562041 A
- US 3733238 A
- US 5110403 A
- US 6036796 A
- US 6508641 B
- US 6645330 B
- US 4854984 A
- US 6291039 B
- US 6248195 B
- US 8778127 B
- US 9005392 B
- US 20140377513 A1
- US 20140377506 A1
- US 20180168878 A1
- US 20180168877 A1
- US 20180168880 A1
- US 20180170027 A1
- US 20180169964 A1
- US 20180168879 A1
- US 20180170026 A1
- US 20180168889 A1
- US 20180168874 A1
- US 20180168875 A1
- US 20180168890 A1
- US 20180168887 A1
- US 20180168892 A1
- US 20180168876 A1
- US 20180168891 A1
- US 20190070042 A1
- US 20190070041 A1
- US 62989059
- US 62984837 B
- US 20190298586 A1

- US 62984837